(19) Europäisches Patentamt
European Patent Office
Office européen des brevets

(11) EP 1 565 512 B1

(12) **EUROPÄISCHE PATENTSCHRIFT**

(45) Veröffentlichungstag und Bekanntmachung des Hinweises auf die Patenterteilung:
**04.07.2018 Patentblatt 2018/27**

(21) Anmeldenummer: **03810455.0**

(22) Anmeldetag: **31.10.2003**

(51) Int Cl.:
***C08G 77/54*** *(2006.01)* ***D06M 15/643*** *(2006.01)*

(86) Internationale Anmeldenummer:
**PCT/EP2003/050775**

(87) Internationale Veröffentlichungsnummer:
**WO 2004/041912 (21.05.2004 Gazette 2004/21)**

(54) **LINEARE POLYAMINO- UND/ODER POLYAMMONIUM-POLYSILOXANCOPOLYMERE I**

LINEAR POLYAMINO AND/OR POLYAMMONIUM POLYSILOXANE COPOLYMERS I

COPOLYMÈRES POLYAMINO-POLYSILOXANE ET/OU POLYAMMONIUM-POLYSILOXANE LINÉAIRES I

(84) Benannte Vertragsstaaten:
**AT BE BG CH CY CZ DE DK EE ES FI FR GB GR HU IE IT LI LU MC NL PT RO SE SI SK TR**

(30) Priorität: **04.11.2002 DE 10251526**

(43) Veröffentlichungstag der Anmeldung:
**24.08.2005 Patentblatt 2005/34**

(73) Patentinhaber: **Momentive Performance Materials GmbH**
**51368 Leverkusen (DE)**

(72) Erfinder:
- **LANGE, Horst**
**44879 Bochum (DE)**
- **WITOSSEK, Anita**
**40764 Langenfeld (DE)**
- **WAGNER, Roland**
**53757 Bonn (DE)**
- **STACHULLA, Karl-Heinz**
**51370 Leverkusen (DE)**
- **Graydon, Andrew Russell**
**Tyne and Wear NE8 1TP (GB)**
- **HARTSHORN, Richard, Timothy**
**Cincinnati, OH 45208 (US)**
- **BOUTIQUE, Jean-Pol**
**B-5030 Gembloux (BE)**
- **DELPLANQUE, Patrick, Firmin, August**
**B-9270 Laarne (BE)**
- **JOHNSTON, James, Pyott**
**B-1785 Merchtem (BE)**
- **SOCKEL, Karl-Heinz**
**51373 Leverkusen (DE)**

(74) Vertreter: **Gille Hrabal**
**Brucknerstrasse 20**
**40593 Düsseldorf (DE)**

(56) Entgegenhaltungen:
**WO-A-02/10256** **WO-A-02/10257**
**WO-A-02/10259** **WO-A1-99/32539**
**US-A- 4 891 166** **US-A- 5 098 979**
**US-A- 5 153 294** **US-B1- 6 240 929**
**US-B1- 6 242 554**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 565 512 B1

## Beschreibung

Die Erfindung betrifft lineare Polyamino- und/oder Polyammonium-Polysiloxancopolymere, insbesondere hydrophile polyquaternäre Polysiloxancopolymere und deren Verwendung als waschbeständige hydrophile Weichmacher.

Aminogruppen-enthaltende Polysiloxane sind als textile Weichmacher bekannt (EP 441530). Die Einführung von durch Ethylenoxid-/Propylenoxideinheiten modifizierten Aminostrukturen als Seitenketten bewirkt eine Verbesserung des Effekts (US 5,591,880, US 5,650,529). Die Alkylenoxideinheiten erlauben hierbei die gezielte Einstellung der hydrophilen-hydrophoben Balance. Nachteilig ist vom synthetischen Standpunkt aus die in die Synthesestrategie eingeschlossene schwierige Veresterung von Aminoalkoholen mit siloxangebundenen Carbonsäuregruppen und bezüglich der weichmachenden Eigenschaften die generelle Kammstruktur der Produkte.

Zur Beseitigung dieser Nachteile ist vorgeschlagen worden, $\alpha,\omega$-epoxymodifizierte Siloxane mit $\alpha,\omega$-aminofunktionalisierten Alkylenoxiden umzusetzen, und diese Produkte als hydrophile Weichmacher einzusetzen (US 5,807,956, US 5,981,681).

Zur Verbesserung der Substantivität sind Versuche unternommen worden, quartäre Ammoniumgruppen in alkylenoxidmodifizierte Siloxane einzuführen. Verzweigte alkylenoxidmodifizierte Polysiloxanquats ("Polysiloxanquats" sind Polydiorganosiloxan-Polyalkylammonium-Verbindungen) sind aus $\alpha,\omega$-OH terminierten Polysiloxanen und Trialkoxysilanen durch Kondensation synthetisiert worden. Die quartäre Ammoniumstruktur wird über das Silan eingebracht, wobei das quartäre Stickstoffatom durch Alkylenoxideinheiten substituiert ist (US 5,602,224).

Streng kammartige alkylenoxidmodifizierte Polysiloxanquats sind ebenfalls beschrieben worden (US 5,098,979). Die Hydroxylgruppen von kammartig substituierten Polyethersiloxanen werden mit Epichlorhydrin in die entsprechenden Chlorhydrinderivate überführt. Anschließend erfolgt eine Quaternierung mit tertiären Aminen. Aus diesem Grund heraus sind die Hydroxylgruppen kammartig substituierter Polyethersiloxane alternativ mit Chloressigsäure verestert worden. Durch die Carbonylaktivierung kann die abschließende Quaternierung erleichtert vollzogen werden (US 5,153,294, US 5,166,297).

In US 6,242,554 werden $\alpha,\omega$-difunktionelle Siloxanderivate beschrieben, die jeweils über eine separate quartäre Ammonium- und Alkylenoxideinheit verfügen. Diese Materialien zeichnen sich durch eine verbesserte Kompatibilität zu polaren Umgebungen aus.

Die Reaktion von $\alpha,\omega$-Diepoxiden mit tertiären Aminen in Gegenwart von Säuren liefert $\alpha,\omega$-diquartäre Siloxane, welche zu Haarpflegezwecken eingesetzt werden können (DE-PS 37,19,086). Neben tetraalkylsubstituierten quartären Ammoniumstrukturen werden auch aromatische Imidazoliniumderivate beansprucht.

Eine Verringerung der Auswaschbarkeit aus Haaren kann erzielt werden, wenn die $\alpha,\omega$-Diepoxide mit di-tertiären Aminen in Gegenwart von Säuren zu langkettigen polyquartären Polysiloxanen umgesetzt werden (EP 282720). Aromatische quartäre Ammoniumstrukturen werden nicht offenbart. Derartige Derivate werden in US 6,240,929 behandelt. In einem ersten Schritt werden hierzu aus Imidazol und geeigneten difunktionellen Alkylierungsagenzien zwei Imidazoleinheiten aufweisende Diamine synthetisiert, welche nachfolgend in einer zur EP 282720 analogen Weise in polyquaternäre Polysiloxane überführt werden. Auf diese Weise hergestellte kationische Verbindungen sollen eine weiter erhöhte Kompatibilität gegenüber den in kosmetischen Formulierungen vorhandenen anionischen Tensiden besitzen.

Allerdings bezieht sich die Auswaschbeständigkeit aus Haaren auf den kurzzeitigen Angriff von vornehmlich Wasser und sehr milden, die Haut nicht irritierenden Tensiden, während waschbeständige, hydrophile Weichmacher für Textilien dem Angriff konzentrierter Tensidlösungen mit hohem Fett- und Schmutzlösevermögen zu widerstehen haben. Erschwerend kommt hinzu, daß moderne Waschmittel stark alkalische Komplexbildner, oxydativ wirkende Bleichmittel und komplexe Enzymsysteme enthalten und die Fasern der Einwirkung oftmals über Stunden bei erhöhten Temperaturen ausgesetzt sind. Aus der WO 02/10259 sind polyquaternäre Polysiloxanverbindungen bekannt, in denen zusätzlich hydrophile Einheiten (EO-Einheiten) eingebaut sind sowie die Anordnung und Sequenzfolge der Quateinheiten zu hydrophilen Einheiten derart verändert werden kann, dass in der Folge ein besserer hydrophiler Weichgriff ohne Verlust der Substantivität auf z.B. Textilien (Baumwolle, Polyester) erzielbar wird.

Weitere Ansätze zur Verbesserung der Kompatibilität mit anionischen Tensidsystemen bzw. der Effizienz der Siloxanabscheidung auf Oberflächen zielen ab auf die Verwendung größerer Mengen kationischer Tenside (WO 00/71806 und WO 00/71807) oder die Nutzung kationischer Polysaccharidderivate (J.V.Gruber et. al., Colloids and Surfaces B: Biointerfaces 19 (2000) 127 - 135) in Mischungen mit Polydimethylsiloxanen.

Hoch geladene, sehr hydrophile synthetische Polykationics sind ebenfalls in der Lage, die Kompatibilität mit anionischen Tensidsystemen zu verbessern (US 6,211,139) oder in Gegenwart von Lösungen anionischer Tenside mit Fasern zu assoziieren (WO 99/14300). In der letztgenannten Schrift werden u.a. Polyimidazoliniumderivate beschrieben.

Keiner der behandelten Vorschläge stellt eine befriedigende Lösung für das Problem dar, den durch Silicone möglichen weichen Griff und die ausgeprägte Hydrophilie nach Erstausrüstung eines Textilmaterials auch dann zu erhalten, wenn dieses dem Angriff aggressiver Detergenzienformulierungen im Verlauf wiederholter Waschprozesse bei gegebenenfalls erhöhter Temperatur ausgesetzt wird.

Ein grundsätzlich anderer Ansatz wird in DE-OS 32 36 466 beschrieben. Die Umsetzung von OH-terminierten

Siloxanen mit quartäre Ammoniumstrukturen enthaltenden Alkoxysilanen liefert reaktive Zwischenprodukte, die mit geeigneten Vernetzungsagenzien, wie Trialkoxysilanen, auf der Faseroberfläche zu waschbeständigen Schichten vernetzen sollen. Entscheidender Nachteil dieses Ansatzes ist, daß die über Stunden notwendige Stabilität eines wässrigen Ausrüstungsbades nicht garantiert werden kann und unvorhergesehene Vernetzungsreaktionen im Bad bereits vor der Textilausrüstung auftreten können.

Aus der WO 02/10257 sind Polysiloxan-Verbindungen mit quaternären Ammoniumgruppen bekannt, die aus Diaminen, Polydiorganosiloxangruppenenthaltenden Diepoxiden und Di(halogenalkyl)-esterpolyether-Verbindungen aufgebaut werden. Die Hydrophilie dieser Polysiloxan-Verbindungen ist jedoch nicht immer zufriedenstellend mithin verbesserungswürdig. Der Versuch die Hydrophilie durch eine Kettenverlängerung des Polyetheranteils zu erhöhen, geht jedoch einher mit einer Verringerung des Gewichtsanteils des Polydimethylsiloxanblocks. Dies resultiert wiederum in einer Verringerung des Weichgriffs. Auch eine Erhöhung des Anteils der quaternären Ammoniumgruppen pro Formelgewicht der Wiederholungseinheit geht mit einer Verringerung des Weichgriffs einher.

Keine der zitierten Lösungen lehrt daher, wie eine weitere Erhöhung der Hydrophilie und der Substantivität unter Beibehaltung des Weichgriffes erreicht werden kann, oder wie sich insbesondere diese Eigenschaften für bestimmte Anwendungen quasi maßschneidern lassen.

Es ist somit Aufgabe der Erfindung, lineare Polysiloxancopolymere, deren Herstellung und deren Verwendung als waschbeständige hydrophile Weichmacher bereitzustellen, wobei die linearen Polysiloxancopolymere den Textilien nach entsprechender Applikation einen silicontypischen weichen Griff und eine ausgeprägte Hydrophilie verleihen und dieses Eigenschaftsbild auch nach Einwirkung von Detergenzienformulierungen während wiederholter Waschprozesse bei ggf. erhöhter Temperatur nicht verloren geht. Es ist eine weitere Aufgabe der Erfindung, die Verwendung dieser linearen Polysiloxancopolymere als separate Weichmacher nach der Wäsche von Fasern und/oder Textilien bzw. als Weichmacher in der Wäsche mit auf nichtionogenen oder anionischen/nichtionogenen Tensiden beruhenden Formulierungen bereitzustellen. Ferner sollen die lineare Polysiloxancopolymere Textilverknitterungen verhindern bzw. rückgängig machen. Schließlich besteht die Aufgabe der vorliegenden Erfindung darin ein lineares Polysiloxancopolymer bereitzustellen, dessen Eigenschaften hinsichtlich Weichgriff, Substantivität, Hydrophilie oder dergleichen sich für eine jeweilige Anwendung in einfacher Weise, maßschneidern lässt.

Die vorliegende Erfindung stellt somit lineare Polyamino- und/oder Polyammonium-Polysiloxancopolymere mit den Wiederholungseinheiten:

$$-[V^{2*}-Z^{2}-V^{2*}-Q]- \text{ und } -[V^{1}-Q]-$$

bereit, worin Q aus der Gruppe ausgewählt wird, die besteht aus:

-NR-,

$-N^{+}R_{2}-$

einem gesättigten oder ungesättigten diaminofunktionellen Heterocyclus der Formeln:

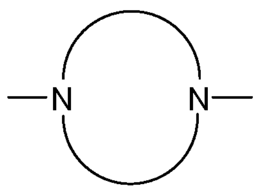

,

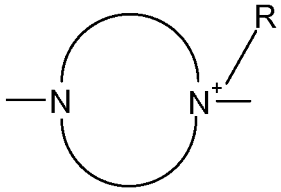

und

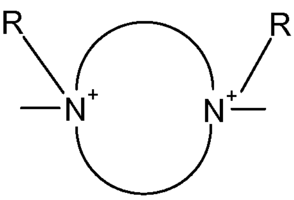

,

sowie

einem aromatischen diaminofunktionellen Heterocyclus der Formel:

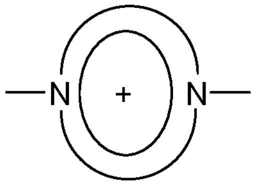

einem dreiwertigen Rest der Formel:

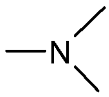

oder

einem dreiwertigen Rest der Formel:

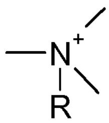

,

worin R jeweils Wasserstoff oder einen einwertigen organischen Rest darstellt,

wobei Q nicht an ein Carbonylkohlenstoffatom bindet,

worin

$V^{2*}$ einen zweiwertigen geradkettigen, cyclischen oder verzweigten, gesättigten, ungesättigten oder aromatischen Kohlenwasserstoffrest mit bis zu 40 Kohlenstoffatomen darstellt, der gegebenenfalls eine oder mehrere Gruppen, ausgewählt aus -O-, -CONH-, -CONR$^2$-, -C(O)- und - C(S)- enthalten kann, und der Rest $V^{2*}$ gegebenenfalls durch eine oder mehrere Hydroxylgruppen substituiert sein kann,

worin $R^2$ = Wasserstoff, ein einwertiger geradkettiger, cyclischer oder verzweigter, gesättigter, ungesättigter $C_1$ bis $C_{16}$ Kohlenwasserstoffrest oder aromatischer $C_6$ bis $C_{16}$ Kohlenwasserstoffrest ist, der eine oder mehrere Gruppen ausgewählt aus -O-, -NH-, -C(O)-, -C(S)- enthalten kann, und der gegebenenfalls durch eine oder mehrere Hydroxylgruppe substituiert sein kann, wobei wenn mehrere Gruppen -NR$^2$ vorliegen, diese gleich oder verschieden sein können,

-$Z^2$- die Formel

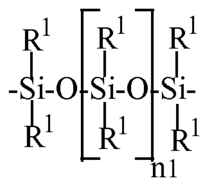

aufweist, worin

$R^1$ gleich oder verschieden sein kann und aus der Gruppe ausgewählt wird, die besteht aus: $C_1$ bis $C_{22}$ Alkyl, Fluor($C_1$-$C_{10}$)alkyl und $C_6$-$C_{10}$ Aryl, und

$n_1$ = 20 bis 1000 bedeutet,

$V^1$ ausgewählt wird aus zweiwertigen oder dreiwertigen, geradkettigen, cyclischen oder verzweigten, gesättigten, ungesättigten oder aromatischen Kohlenwasserstoffresten mit bis zu 1000 Kohlenstoffatomen, die gegebenenfalls eine oder mehrere Gruppen, ausgewählt aus

-O-, -CONH-,

-$CONR^2$-, worin $R^2$ wie oben definiert ist, wobei die Gruppen $R^2$ in den Gruppen $V^1$ und $V^{2*}$-$Z^2$-$V^{2*}$ gleich oder verschieden sein können,

-C(O)-, -C(S)- und -$Z^1$- enthalten können, worin -$Z^1$- eine Gruppe der Formel

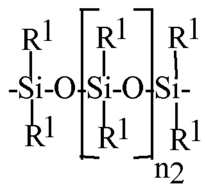

ist, worin

$R^1$ wie oben definiert ist, wobei die Gruppen $R^1$ in den Gruppen $V^1$ und $V^{2*}$-$Z^2$-$V^{2*}$ gleich oder verschieden sein können, und

$n_2$ = 0 bis 19 bedeutet,

und der Rest $V^1$ gegebenenfalls durch eine oder mehrere Hydroxylgruppen substituiert sein kann,

mit der Maßgabe, dass die dreiwertigen Reste Q und die dreiwertigen Reste $V^1$ ausschließlich der Absättigung untereinander innerhalb der linearen Hauptkette der Polysiloxan-Copolymere dienen,

und dass in dem Copolymer das molare Verhältnis von

$$-[V^{2}*-Z^{2}-V^{2}*-Q]- \text{ und } -[V^{1}-Q]- < 1:3$$

ist,

und worin die aus den Ammoniumgruppen resultierenden positiven Ladungen durch organische oder anorganische Säureanionen neutralisiert sind,

und deren Säureadditionssalze.

In einer bevorzugten Ausführungsform der Erfindung wird Q aus der Gruppe ausgewählt wird, die besteht aus:

-NR-,
$-N^+R_2-$
einem gesättigten oder ungesättigten diaminofunktionellen Heterocyclus der Formeln:

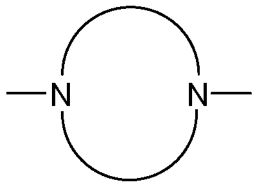

,

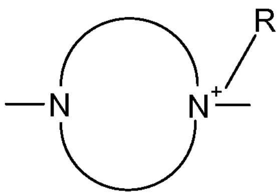

und

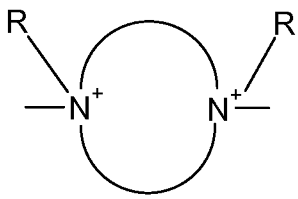

,

sowie
einem aromatischen diaminofunktionellen Heterocyclus der Formel:

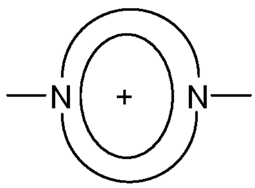

worin R wie oben definiert ist, und $V^1$ und $V^{2*}-Z^2-V^{2*}-$ sind zweiwertige Reste.

In einer weiteren bevorzugten Ausführungsform der Erfindung wird Q aus der Gruppe ausgewählt wird, die besteht aus:

Einer Aminoeinheit der Formel:

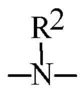

einer Ammoniumeinheit der Formel:

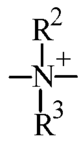

einer quaternierten Imidazoleinheit der Struktur

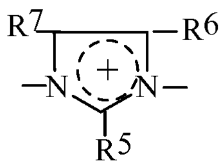

,

einer quaternierten Pyrazoleinheit der Struktur

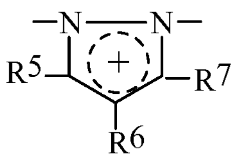

einer zweifach quaternierten Piperazineinheit der Struktur

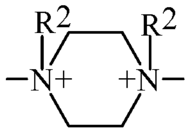

,

einer monoquaternierten Piperazineinheit der Struktur

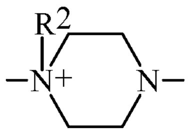

,

einer monoquaternierten Piperazineinheit der Struktur

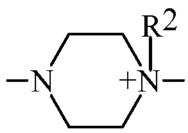

,

einer zweifach quaternierten Einheit der Struktur

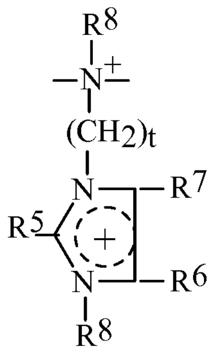

,

einer monoquaternierten Einheit der Struktur

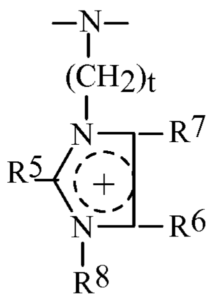

,

einer monoquaternierten Einheit der Struktur

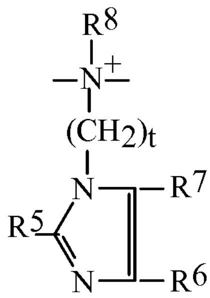

,

einer zweifach quaternierte Einheit der Struktur

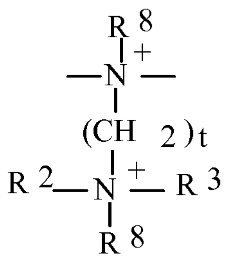

,

einer monoquaternierten Einheit der Struktur

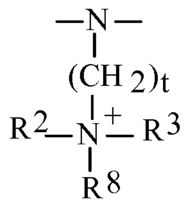

einer monoquaternierten Einheit der Struktur

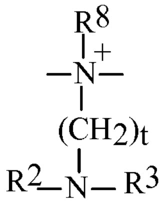

worin

t von 2 bis 10 ist,

$R^2$ wie oben definiert ist, und die Bedeutung von $R^2$ von der Bedeutung der obigen Gruppe $R^2$ gleich oder verschieden sein kann,

$R^3$ die Bedeutung von $R^2$ aufweist, wobei $R^2$ und $R^3$ gleich oder verschieden sein können, oder

$R^2$ und $R^3$ gemeinsam mit dem positiv geladenen Stickstoffatom einen fünf- bis siebengliedrigen Heterocyclus bilden, der gegebenenfalls zusätzlich ein oder mehrere Stickstoff-, Sauerstoff- und/oder Schwefelatome aufweisen kann,

$R^5$, $R^6$, $R^7$ gleich oder verschieden sein können und aus der Gruppe ausgewählt werden, die besteht aus: H, Halogen, Hydroxylgruppe, Nitrogruppe, Cyanogruppe, Thiolgruppe, Carboxylgruppe, Alkylgruppe, Monohydroxyalkylgruppe, Polyhydroxyalkylgruppe, Thioalkylgruppe, Cyanoalkylgruppe, Alkoxygruppe, Acylgruppe, Acetyloxygruppe, Cycloalkylgruppe, Arylgruppe, Alkylarylgruppe, und Gruppen des Typs $-NHR^W$, in denen $R^W$ H, Alkylgruppe, Monohydroxyalkylgruppe, Polyhydroxyalkylgruppe, Acetylgruppe, Ureidogruppe bedeuten, und jeweils zwei der benachbarten Reste $R^5$, $R^6$ und $R^7$ mit den sie an den Heterocyclus bindenden Kohlenstoffatomen aromatische Fünf- bis Siebenringe bilden können, und

$R^8$ die Bedeutung von $R^2$ aufweist, wobei $R^8$ und $R^2$ gleich oder verschieden sein können.

Im Falle, dass Q einen dreiwertigen Rest der Formeln

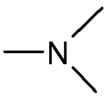
oder
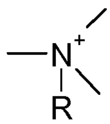

darstellt, dienen diese Reste nicht der Verzweigung der Polysiloxan-Copolymere sondern diese Reste sind ausschließlich mit dreiwertigen Resten $V^1$ verbunden, wobei cyclische Strukturen ausgebildet werden, die Bestandteil der linearen Hauptkette sind, wie z.B. ein Strukturelement der Formel

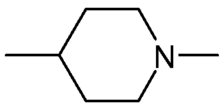
.

Gleichfalls dienen die dreiwertigen Reste $V^1$ nicht der Verzweigung der linearen Polysiloxan-Copolymere.

In der eingangs erwähnten, in Anspruch 1 beanspruchten Ausführungsform weist das erfindungsgemäße lineare Polysiloxancopolymer die folgenden Wiederholungseinheiten auf:

$$-[V^{2*}\text{-}Z^{2}\text{-}V^{2*}\text{-}Q]\text{- und -}[V^{1}\text{-}Q]\text{-}.$$

.

Das molare Verhältnis der Wiederholungseinheiten $-[V^{2*}-Z^2-V^{2*}-Q]-$ zu $-[V^1-Q]-$ entspricht somit dem Verhältnis $V^{2*}-Z^2-V^{2*}/V^1 < 1 : 3$.

Aufgrund dieser molaren Verhältnisse enthalten die erfindungsgemäßen linearen Polysiloxancopolymere zwingend Blöcke, die mehr als eine $-[V^1-Q]$-Einheit, miteinander verknüpft enthalten.

Wie weiter unten im Zusammenhang mit dem erfindungsgemäßen Verfahren zur Herstellung der lineare Polysiloxancopolymere der Erfindung noch ausführlich erläutert wird, können die blockartigen Sequenzen, die mehr als eine $-[V^1-Q]$-Einheit miteinander verknüpft aufweisen, je nach Herstellweise regelmäßig mit den $V^{2*}-Z^2-V^{2*}-Q-$-Einheiten oder unregelmäßig mit den $V^{2*}-Z^2-V^{2*}-Q$-Einheiten verbunden werden.

Dies meint folgendes:

Bei der regelmäßigen Verbindung, bei der beispielsweise ein der Gruppe $-Q-[V^1-Q]_x$-entsprechendes Präpolymer mit $V^{2*}-Z^2-V^{2*}-$ entsprechenden Monomer-Einheiten im molaren Verhältnis 1:1 umgesetzt wird, lassen sich die linearen Polysiloxancopolymere wie folgt darstellen:

$$-\{V^{2*}\text{-}Z^{2}\text{-}V^{2*}\text{--}Q\text{-}[V^{1}\text{-}Q]_{x}\text{-}\}\text{-}.$$

x kann dabei 2 bis 2000 sein und ist der Mittelwert. Die durch die Formel $-\{V^{2*}-Z^2-V^{2*}--Q-[V^1-Q]_x-\}-$ dargestellten linearen Polysiloxancopolymere sind dadurch gekennzeichnet, dass sie im wesentlichen keine miteinander verknüpften $-V^{2*}-Z^2-V^{2*}-Q$-Einheiten aufweisen, oder mit anderen Worten, sind zwei $-V^{2*}-Z^2-V^{2*}-Q$-Einheiten stets durch mindestens eine $-V^1-Q$-Einheit unterbrochen.

Bei der unregelmäßigen Verbindung, bei der beispielsweise Q-Einheiten entsprechende Monomere mit $V^1$ entsprechenden Monomer-Einheiten und $V^{2*}-Z^2-V^{2*}$ entsprechenden Monomer-Einheiten im Verhältnis $Q/(V^1 + V^{2*}-Z^2-V^{2*})$, mit $V^{2*}-Z^2-V^{2*}/V^1 < 1 : 3$, von 1:1 umgesetzt wird, lassen sich die linearen Polysiloxancopolymere wie folgt darstellen:

$$-Q\text{-}(V^{1},\ V^{2*}\text{-}Z^{2}\text{-}V^{2*})\text{-},$$

worin das Verhältnis $V^{2*}-Z^2-V^{2*}/V^1 < 1 : 3$ ist. Dabei sind die Gruppen $V^1$ und $V^{2*}-Z^2-V^{2*}$ statistisch über die Copolymerkette verteilt. Im Unterschied zu dem durch die regelmäßige Verbindung hergestellten linearen Polysiloxancopolymere kann dieses Copolymer auch benachbarte $-Q-V^{2*}-Z^2-V^{2*}-$-Einheiten aufweisen. In einer weiteren bevorzugten Ausführungsform der vorliegenden Erfindung wird die Gruppe $V^1$ ausgewählt aus zweiwertigen, geradkettigen, cyclischen oder verzweigten, gesättigten, ungesättigten oder aromatischen Kohlenwasserstoffresten mit bis zu 400 Kohlenstoffatomen, die gegebenenfalls eine oder mehrere Gruppen, ausgewählt aus

-O-, -CONH-, $-CONR^2-$, worin $R^2$ wie oben definiert ist, -C(O)-, -C(S)- und $-Z^1-$ enthalten kann, worin $-Z^1-$ eine Gruppe der Formel

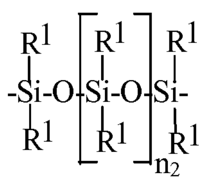

ist, worin

$R^1$ $C_1$ bis $C_3$ Alkyl, Fluor($C_3$-$C_6$)alkyl oder $C_6$- Aryl ist, und $n_2$ wie oben definiert ist.

In einer weiteren bevorzugten Ausführungsform der vorliegenden Erfindung wird die Gruppe Q ausgewählt aus:

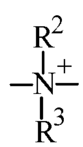

einer quaternierten Imidazoleinheit der Struktur

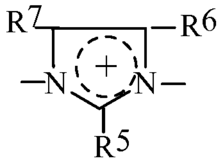

,

einer quaternierten Pyrazoleinheit der Struktur

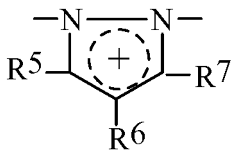

einer zweifach quaternierten Piperazineinheit der Struktur

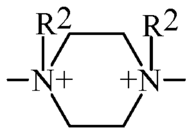

,

einer monoquaternierten Piperazineinheit der Struktur

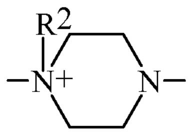

,

einer monoquaternierten Piperazineinheit der Struktur

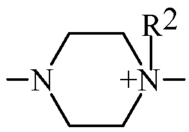

,

einer monoquaternierten Einheit der Struktur

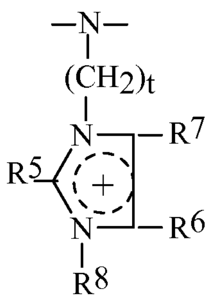

,

worin $R^2$, $R^3$, $R^4$, $R^5$, $R^6$, $R^7$ und $R^8$ wie oben definiert sind.

In einer weiteren bevorzugten Ausführungsform der vorliegenden Erfindung erfüllt das molare Verhältnis -[$V^{2*}$-$Z^2$-$V^{2*}$]- /$V^1$ die Beziehung

$$0{,}0005 < -[V^{2}*-Z^{2}-V^{2}*]- /V^{1} < 0{,}33, (= 3 < V^{1}/-[V^{2}*-Z^{2}-V^{2}*]- < 2000)$$

bevorzugter die Beziehung

$$0{,}005 < -[V^{2}*-Z^{2}-V^{2}*]- /V^{1} < 0{,}25, (= 4 < V^{1}/-[V^{2}*-Z^{2}-V^{2}*]- < 200)$$

noch bevorzugter die Beziehung

$$0{,}01 < -[V^{2}*-Z^{2}-V^{2}*]- /V^{1} < 0{,}2 (= 5 < V^{1}/-[V^{2}*-Z^{2}-V^{2}*]- < 100).$$

Bevorzugt sind:

$R^1$ = $C_1$ bis $C_{18}$ Alkyl, insbesondere Methyl, Ethyl, Perfluoralkylethylen, wie Trifluorpropyl und Phenyl,
$n_1$ = 20 bis 400, besonders bevorzugt 20 bis 300, speziell 20 bis 200. In einer weiteren bevorzugten Ausführungsform ist $n_1$ zwischen 20 und 50 oder zwischen 80 und 200. Die Zahl $n_1$ ist dermittlere Polymerisationsgrad der Diorganosiloxy-Einheiten in der Gruppe $Z^2$.
$n_2$ = 0 bis 15, besonders bevorzugt 0 bis 10, speziell 0 bis 5, spezieller 0. Die Zahl $n_2$ ist der mittlere Polymerisationsgrad aus Mn der Diorganosiloxy-Einheiten in der Gruppe $Z^1$.
$V^{2*}$ = ein zweiwertiger geradkettiger, cyclischer oder verzweigter, gesättigter, ungesättigter $C_3$ bis $C_{16}$ Kohlenwasserstoffrest oder aromatischer $C_8$ bis $C_{20}$ Kohlenwasserstoffrest, der gegebenenfalls eine oder mehrere Gruppen, ausgewählt aus -O-, -CONH-, $-CONR^2-$, -C(O)-, -C(S)- enthalten kann und durch eine oder mehrere OH-Gruppe substituiert sein kann, worin
$R^2$ = Wasserstoff, ein einwertiger geradkettiger, cyclischer oder verzweigter, gesättigter, ungesättigter $C_1$ bis $C_{16}$ Kohlenwasserstoffrest oder aromatischer $C_6$ bis $C_{16}$ Kohlenwasserstoffrest ist, der eine oder mehrere Gruppen ausgewählt aus -O-, -NH-, -C(O)-, -C(S)- enthalten kann, und der gegebenenfalls durch eine oder mehrere Hydroxylgruppe substituiert sein kann, wobei wenn mehrere Gruppen $-NR^2$ vorliegen, diese gleich oder verschieden sein können,
Q =

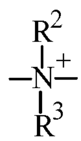

eine quaternierte Imidazoleinheit der Struktur

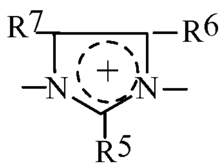

,

eine zweifach quaternierte Piperazineinheit der Struktur

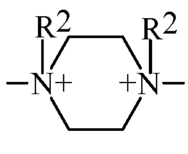

,

eine monoquaternierte Piperazineinheit der Struktur

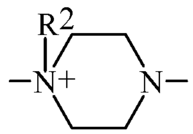

,

eine monoquaternierte Piperazineinheit der Struktur

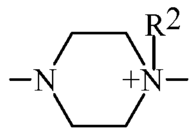

,

eine monoquaternierte Einheit der Struktur

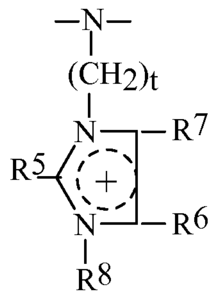

,

worin $R^2$, $R^3$, $R^4$, $R^5$, $R^6$, $R^7$ und $R^8$ wie oben definiert sind.

Besonders bevorzugt steht $V^{2*}$ für einen zweiwertigen geradkettigen, cyclischen oder verzweigten, gesättigten, ungesättigten oder aromatischen Kohlenwasserstoffrest mit bis zu 16 Kohlenstoffatomen, der eine oder mehrere Gruppen, ausgewählt aus -O-, -CONH-, $-CONR^2-$, worin $R^2$ wie oben definiert ist, -C(O)-, -C(S)- enthalten kann und mit einer oder mehreren Hydroxylgruppen substituiert sein kann. Noch bevorzugter wird $-V^{2*}-$ ausgewählt aus Gruppen der Formeln:

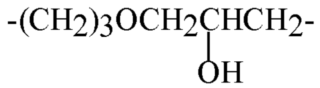

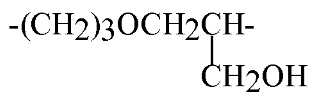

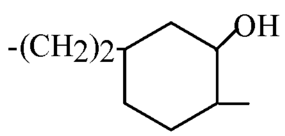

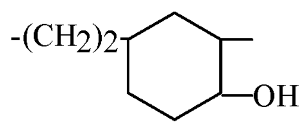

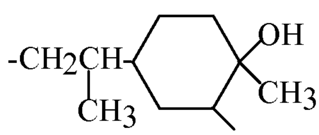

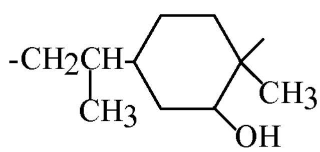

$-(CH_2)_2-$, $-(CH_2)_3-$ , $-(CH_2)_4-$, $-(CH_2)_5-$, $-(CH_2)_6-$,

$-CH=CHCH_2-$, $-CH=CHCH_2CH_2-$,

$-CH_2CH_2CH_2OC(O)CH_2-$, $-CH_2CH_2CH_2OC(O)CH_2CH_2-$,

$-CH{=}CHCH_2OC(O)CH_2-$, $-CH{=}CHCH_2OC(O)CH_2CH_2-$,

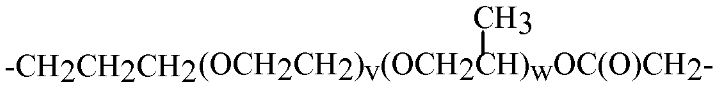

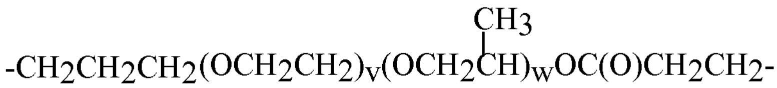

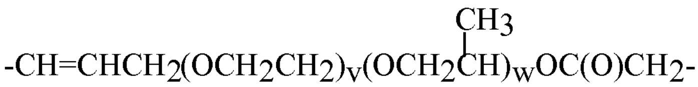

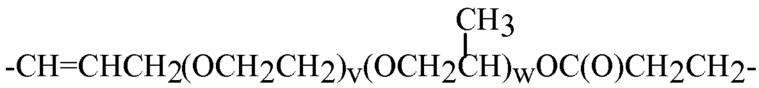

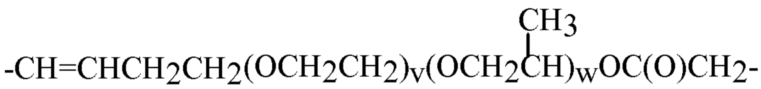

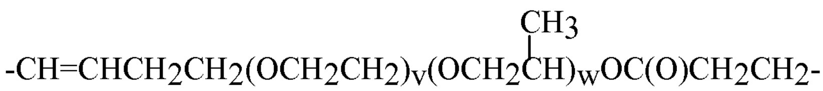

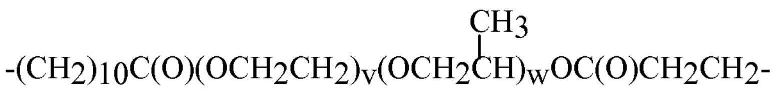

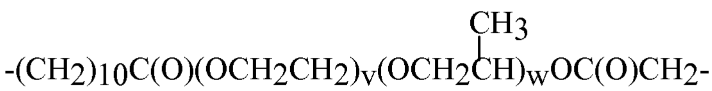

mit $v+w \geq 0$,

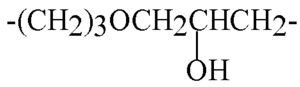

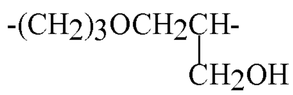

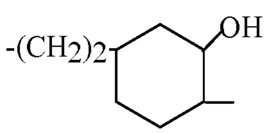

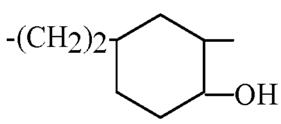

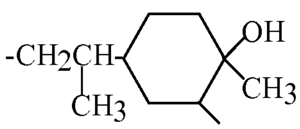

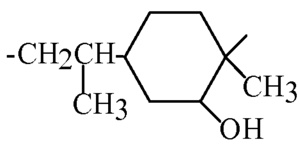

$-(CH_2)_3-$, $-(CH_2)_4-$, $-(CH_2)_5-$, $-(CH_2)_6-$,

$-CH{=}CHCH_2-$, $-CH{=}CHCH_2CH_2-$,

$-CH_2CH_2CH_2OC(O)CH_2-$, $-CH_2CH_2CH_2OC(O)CH_2CH_2-$,

$R^2$ steht bevorzugt für:
Wasserstoff, $-CH_3$, $-CH_2CH_3$, $-(CH_2)_2CH_3$, $-(CH_2)_3CH_3$, $-(CH_2)_5CH_3$, $-CH_2CH_2OH$,

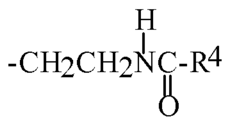 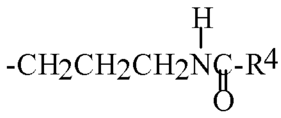

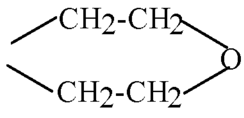 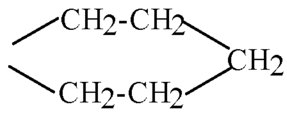

mit
$R^4$ = geradkettiger, cyclischer oder verzweigter $C_1$ bis $C_{18}$ Kohlenwasserstoffrest, der durch eine oder mehrere Gruppen, ausgewählt aus -O-, -NH-, -C(O)-, und - C(S)- enthalten kann und durch eine oder mehrere OH-Gruppen substituiert sein kann, speziell unsubstituierte $C_5$ bis $C_{17}$ Kohlenwasserstoffreste, die sich von den entsprechenden Fettäuren ableiten oder aber hydroxylierte $C_3$ bis $C_{17}$ Reste, die auf hydroxylierte Carbonsäuren, speziell Saccharidcarbonsäuren zurückgeführt werden können und ganz speziell

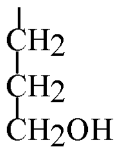 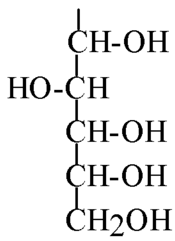 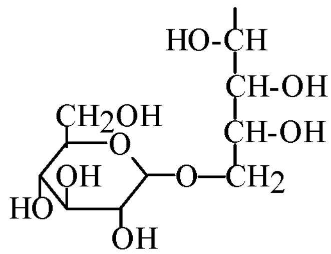

bedeuten.
$V^1$ steht bevorzugt für

- $-R^9-$, worin $R^9$ einen zweiwertigen, gesättigten oder einfach oder mehrfach ungesättigten, geradkettigen oder verzweigten Kohlenwasserstoffrest mit zwei bis 25 Kohlenstoffatomen darstellt,
- $-(CH_2)_uC(O)O-[(CH_2CH_2O)_q-(CH_2CH(CH_3)O)_r]-C(O)(CH_2)_u-$
- $-(CH_2)_uC(O)O-R^9-O-C(O)(CH_2)_u-$, worin $R^9$ wie zuvor definiert ist,
- $-(CH_2)_u-R^{10}-(CH_2)_u-$, worin $R^{10}$ eine aromatische Gruppe ist,
- $-[CH_2CH_2O]_q-[CH_2CH(CH_3)O]_r-CH_2CH_2-$,
- $-CH(CH_3)CH_2O[CH_2CH_2O]_q-[CH_2CH(CH_3)O]_r-CH_2CH(CH_3)-$
- $-CH_2CH(OH)CH_2-$,
- $-CH_2CH(OH)(CH_2)_2CH(OH)CH_2-$,
- $-CH_2CH(OH)CH_2OCH_2CH(OH)CH_2OCH_2CH(OH)CH_2-$ und
- $-CH_2CH(OH)CH_2O-[CH_2CH_2O]_q-[CH_2CH(CH_3)O]_r-CH_2CH(OH)CH_2-$

worin
u von 1 bis 3 ist,
q und r von 0 bis 200, bevorzugt von 0 bis 100, bevorzugter von 0 bis 70 und besonders bevorzugt 0 bis 40 ist, und
$q + r > 0$ ist.
Bevorzugte Varianten von $V^1$ sind Strukturen der Formel

$$-CH_2C(O)O-[CH_2CH_2O]_q-[CH_2CH(CH_3)O]_r-C(O)CH_2-,$$

$$-CH_2CH_2C(O)O-[CH_2CH_2O]_q-[CH_2CH(CH_3)O]_r-C(O)CH_2CH_2-,$$

$$-CH_2CH_2CH_2C(O)O-[CH_2CH_2O]_q-[CH_2CH(CH_3)O]_r-C(O)CH_2CH_2CH_2-,$$

veresterte Alkylen-, Alkenylen, Alkinyleneinheiten, speziell der Strukturen

$-CH_2C(O)O-[CH_2]_o-OC(O)CH_2-$,
$-CH_2CH_2C(O)O-[CH_2]_o-OC(O)CH_2CH_2-$,
$-CH_2CH_2CH_2C(O)O-[CH_2]_o-OC(O)CH_2CH_2CH_2-$
$-CH_2C(O)O-CH_2C{\equiv}CCH_2-OC(O)CH_2-$,
$-CH_2CH_2C(O)O-CH_2C{\equiv}CCH_2-OC(O)CH_2CH_2-$,
$-CH_2CH_2CH_2C(O)O-CH_2C{\equiv}CCH_2-OC(O)CH_2CH_2CH_2-$,
$-CH_2C(O)O-CH_2CH=CHCH_2-OC(O)CH_2-$,
$-CH_2CH_2C(O)O-CH_2CH=CHCH_2-OC(O)CH_2CH_2-$,
$-CH_2CH_2CH_2C(O)O-CH_2CH=CHCH_2-OC(O)CH_2CH_2CH_2-$, Alkylen-, Alkenylen-, Alkinylen- und Aryleinheiten, speziell der Strukturen $-[CH_2]_o-$
mit o = 2 bis 6,
$-CH_2C{\equiv}CCH_2-$, $-CH_2CH=CHCH_2-$, $-CH(CH_3)CH_2CH_2-$,

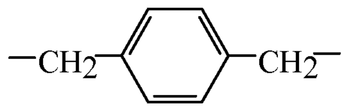

Polyalkylenoxideinheiten, speziell der Strukturen
$-[CH_2CH_2O]_q-[CH_2CH(CH_3)O]_r-CH_2CH_2-$,
$-CH(CH_3)CH_2O[CH_2CH_2O]_q-[CH_2CH(CH_3)O]_r-CH_2CH(CH_3)-$mit
mono-, di- oder polyhydroxyfunktionelle Einheiten, speziell der Strukturen
$-CH_2CH(OH)CH_2-$, $-CH_2CH(OH)(CH_2)_2CH(OH)CH_2-$,
$-CH_2CH(OH)CH_2OCH_2CH(OH)CH_2OCH_2CH(OH)CH_2-$,
$-CH_2CH(OH)CH_2O-[CH_2CH_2O]_q-[CH_2CH(CH_3)O]_r-CH_2CH(OH)CH_2-$
mit
q = 0 bis 200,
r = 0 bis 200

Bevorzugt sind q = 1 bis 50, insbesondere 2 bis 50, speziell 1 bis 20, ganz speziell 1 bis 10, sowie 1 oder 2, r = 0 bis 100, insbesondere 0 bis 50, speziell 0 bis 20, ganz speziell 0 bis 10, sowie 0 oder 1 oder 2.

Die Erfindung betrifft weiterhin ein Verfahren zur Herstellung der erfindungsgemäßen linearen Polysiloxane, worin

a) mindestens eine Aminverbindung, ausgewählt aus einer Diamin-Verbindung und/oder einer primären oder sekundären Monoaminverbindung, mit mindestens zwei difunktionellen, zur Reaktion mit den Aminofunktionen der Amin-Verbindung befähigten organischen Verbindungen umgesetzt werden, wobei das molare Verhältnis der organischen Verbindungen so gewählt wird, dass die Bedingung $-[V^{2*}-Z^2-V^{2*}]/V^1 < 1:3$ erfüllt wird, oder
b) mindestens zwei Mol einer Aminverbindung, ausgewählt aus einer Diamin-Verbindung und/oder einer primären oder sekundären Monoaminverbindung, mit einem Mol einer difunktionellen, zur Reaktion mit den Aminofunktionen der Aminverbindung befähigten organischen Verbindung unter Bildung einer Diaminverbindung (Monomer) umgesetzt wird, die anschließend mit mindestens einer Aminverbindung, ausgewählt aus einer Diamin-Verbindung und/oder einer primären oder sekundären Monoaminverbindung, und mindestens einer weiteren difunktionellen zur Reaktion mit den Aminofunktionen der Aminverbindungen befähigten organischen Verbindung umgesetzt wird, oder
c) eine Aminverbindung, ausgewählt aus einer Diamin-Verbindung und/oder einer primären oder sekundären Monoaminverbindung, mit einer difunktionellen, zur Reaktion mit den Aminofunktionen der Aminverbindungen befähigten organischen Verbindung unter Bildung einer Diaminverbindung (aminoterminiertes Oligomer) umgesetzt wird, die anschließend mit mindestens einer difunktionellen zur Reaktion mit den Aminofunktionen der Diamin-Verbindungen befähigten organischen Verbindung umgesetzt wird, oder
d) eine Aminverbindung, ausgewählt aus einer Diamin-Verbindung und/oder einer primären oder sekundären Monoaminverbindung, mit einer difunktionellen, zur Reaktion mit den Aminofunktionen der Aminverbindung befähigten organischen Verbindung unter Bildung einer difunktionellen, zur Reaktion mit Aminofunktionen befähigten Verbindung (difunktionelles Oligomer) umgesetzt wird, die anschließend mit mindestens einer Aminverbindung, ausgewählt aus einer Diamin-Verbindung und/oder einer primären oder sekundären Monoaminverbindung, und mindestens einer weiteren zur Reaktion mit Aminofunktionen befähigten Verbindung umgesetzt wird,

wobei gegebenenfalls monofunktionelle, bevorzugt tertiäre Monoamine oder geeignete, zur Kettenfortpflanzung nicht befähigte Monoamine und/oder monofunktionelle, zur Reaktion mit Aminofunktionen befähigten Verbindungen als Kettenabbruchsmittel hinzugesetzt werden können, und die Stöchiometrie der Aminofunktionen und der zur Reaktion mit

Aminofunktionen befähigten funktionellen Gruppen in der letzten Stufe der Umsetzung stets etwa 1:1 beträgt, und wobei gegebenenfalls vorhandene Aminofunktionen protoniert, alkyliert oder quaterniert werden können.

Variante a), worin mindestens eine Diamin-Verbindung, ausgewählt aus einer Diamin-Verbindung und/oder einer primären oder sekundären Monoaminverbindung, mit mindestens zwei difunktionellen, zur Reaktion mit den Aminofunktionen der Aminverbindung befähigten organischen Verbindungen umgesetzt werden, wobei das molare Verhältnis der organischen Verbindungen so gewählt wird, dass die Bedingung $-[V^{2*}-Z^2-V^{2*}]- /V^1 < 1 : 3$ erfüllt wird, lässt sich somit schematisch beispielsweise wie folgt darstellen:

$$-[N\text{-}N]- + -[V^1]- + -[V^{2*}\text{-}Z^2\text{-}V^{2*}]- \rightarrow -[Q\text{-}(V^1, V^{2*}\text{-}Z^2\text{-}V^{2*})]-$$

oder

$$-[N]- + -[V^1]- + -[V^{2*}\text{-}Z^2\text{-}V^{2*}]- \rightarrow -[Q\text{-}(V^1, V^{2*}\text{-}Z^2\text{-}V^{2*})]-$$

wobei -[N-N]- ein cyclisches, der Definition von Q entsprechendes Diamin oder ein $V^1$-enthaltendes Diamin $-[N-V^1-N]-$ oder ein $V^{2*}-Z^2-V^{2*}$-enthaltendes Diamin $-[N-V^{2*}-Z^2-V^{2*}]-N]-$ einschließen kann, wobei aus den letzteren jeweils zwei Q-Einheiten und eine $V^1$ bzw. zwei $V^{2*}-Z^2-V^{2*}$-Einheiten hervorgehen, und $-[V^1]-$ und $-[V^{2*}-Z^2-V^{2*}]-$ den Wiederholungseinheiten $V^1$ und $V^{2*}-Z^2-V^{2*}$ entsprechende Monomere darstellen sollen,

und -[N]- ein primäres oder sekundäres zur Kettenfortpflanzung geeignetes Monoamin darstellt,

Aus den -[N-N]- und/oder -[N]-Einheiten wird dabei mindestens eine höher-alkylierte Amin- oder eine quaternäre Ammonium-Einheit Q gebildet, wobei bei der Polymerisation gebildete sekundäre oder tertiäre Aminofunktionen gegebenenfalls nach der Polymerisation in einem separaten Schritt protoniert oder quaterniert werden können. Bevorzugt ist die Bildung quarternärer Ammoniumeinheiten.

Bevorzugte Beispiele von -[N-N]- sind wie unten noch ausführlicher beschrieben wird: Piperazin und Imidazol, bevorzugte Diamin-Einheiten $-[N-V^1-N]-$ schließen beispielsweise ein: Polymethylendiamine, wie Tetramethyl-Hexamethylendiamin, $\alpha,\omega$-diaminoterminierte Polyether, wie z.B. Jeffamine, etc.

Bevorzugte Diamin-Einheiten $-[N-V^{2*}-Z^2-V^{2*}-N]-$ schließen beispielsweise Umsetzungprodukte von $\alpha,\omega$-Dihydrogenpolydialkylsiloxane mit Allylaminen ein.

Bevorzugte Beispiele von -[N]- sind wie unten noch ausführlicher beschrieben z.B. Dimethylamin.

Die Verwendung von Diaminen -[N-N]- ist an sich bevorzugt.

Bevorzugte $-[V^1]-$-Monomere schließen beispielweise Epichlorhydrin, Bis-Chloralkylester, Bisepoxide oder Bisacrylate. Es können bevorzugt auch Mischungen der genannten $-[V^1]-$-Monomere, wie z.B. Mischungen aus Epichlorhydrin, Bis-Chloralkylester oder Bisepoxiden umgesetzt werden. Bevorzugte $-[V^{2*}-Z^2-V^{2*}]-$-Monomere zur Bildung der $V^{2*}-Z^2-V^{2*}$-Wiederholungseinheiten sind insbesondere $\alpha,\omega$-diepoxyterminierte Polydialkylsiloxane.

Variante b) lässt sich sowohl mit Diaminen, -[N-N]-, als auch geeigneten Monoaminen -[N]- durchführen und lässt sich schematisch beispielsweise wie folgt darstellen:

Variante b1)

Schritt 1): 2 $-[N-N]- + -[V^{2*}-Z^2-V^{2*}]-$ oder $-[V^1]- \rightarrow -[N-N-V^1-N-N]-$ oder $-[N-N-V^{2*}-Z^2-V^{2*}-N-N]-$
Schritt 2.1): $-[N-N-V^{2*}-Z^2-V^{2*}-N-N]- + [V^1]- + -[N-N]- \rightarrow$,
Schritt 2.2): $-[N-N-V^1-N-N]- + -[V^{2*}-Z^2-V^{2*}]- + -[N-N]- \rightarrow$,

wobei die Stöchiometrie so gewählt wird, dass die Bedingung $-[V^{2*}-Z^2-V^{2*}]-/V^1<1 : 3$ erfüllt ist.

Bezüglich der bevorzugt verwendeten Monomer-Einheiten -[N-N]-, $-[V^1]-$ und - $[V^{2*}-Z^2-V^{2*}]-$ gilt das für Schritt a) Gesagte.

Variante b2)

Schritt 1):

Schritt 1): 2 $-[N]- + -[V^{2*}-Z^2-V^{2*}]-$ oder $-[V^1]- \rightarrow -[N-V^1-N]-$ oder $-[N-[V^{2*}-Z^2-V^{2*}]-N]-$

Schritt 2.1):

$$-[N-[V^{2*}-Z^2-V^{2*}]-N]- + -[V^1]- + -[N]- \rightarrow,$$

Schritt 2.2):

$$-[N-V^1-N]- + -[V^{2*}-Z^2-V^{2*}]- + -[N]- \rightarrow,$$

wobei diese Variante wie oben erwähnt nur mit primären oder sekundären Monoaminen durchführbar ist und wobei bezüglich der bevorzugt verwendeten Monomer-Einheiten -[N]-, $-[V^1]-$ und $-[V^{2*}-Z^2-V^{2*}]-$ das für Schritt a) Gesagte gilt.

Variante c) lässt sich schematisch beispielsweise wie folgt darstellen:

Variante c1)

Schritt 1):

$$-[N-N]- + -[V^1]- \rightarrow -[N-N-(V^1-N-N)_x]-$$

Schritt 2):

$$-[N-N-(V^1-N-N)_x]- + -[V^{2*}-Z^2-V^{2*}]- \rightarrow$$

wobei bezüglich der bevorzugt verwendeten Monomer-Einheiten -[N-N]-, $-[V^1]-$ und $-[V^{2*}-Z^2-V^{2*}]-$ das für Schritt a) Gesagte gilt.

Variante c2)

Schritt 1):

$$-[N]- + -[V^1]- \rightarrow -[N-(V^1-N)_x]-$$

Schritt 2):

$$-[N-(V^1-N)_x]- + -[V^{2*}-Z^2-V^{2*}]- \rightarrow$$

wobei bezüglich der bevorzugt verwendeten Monomer-Einheiten -[N]-, $-[V^1]-$ und $-[V^{2*}-Z^2-V^{2*}]-$ das für Schritt a) Gesagte gilt.

Variante d) lässt sich schematisch beispielsweise wie folgt darstellen:

Variante d1)

Schritt 1):

$$-[V^1]- + -[N-N]- \rightarrow -[V^1-(N-N-V^1)_x]-$$

Schritt 2):

$$-[V^1-(N-N-V^1)_x]- + -[V^{2*}-Z^2-V^{2*}]- + -[N]- \text{ oder } -[N-N]- \rightarrow$$

wobei bezüglich der bevorzugt verwendeten Monomer-Einheiten -[N-N]-, $-[V^1]-$ und $-[V^{2*}-Z^2-V^{2*}]-$ das für Schritt a) Gesagte gilt.

Variante d2)

Schritt 1):

$$-[V^1]- + -[N]- \rightarrow -[V^1-(N-V^1)_x]-$$

Schritt 2):

$$-[V^1-(N-V^1)_x]- + -[V^{2*}-Z^2-V^{2*}]- + -[N]- \text{ oder } -[N-N]- \rightarrow$$

wobei bezüglich der bevorzugt verwendeten Monomer-Einheiten -[N]-, $-[V^1]-$ und $-[V^{2*}-Z^2-V^{2*}]-$ das für Schritt a) Gesagte gilt.

Für alle oben schematisch dargestellten Varianten gilt, dass auch Mischungen von Monoaminen -[N]- und Diaminen -[N-N]- eingesetzt werden können.

Besonders bevorzugt werden die funktionellen Gruppen der difunktionellen, zur Reaktion mit Aminofunktionen befähigten Verbindungen ausgewählt aus der Gruppe die besteht aus Epoxygruppen und Halogenalkylgruppen.

Als Ausgangspunkt für die Synthesen der erfindungsgemäßen linearen Polysiloxancopolymere sind $\alpha,\omega$ Si-H funktionalisierte Siloxane der allgemeinen Struktur

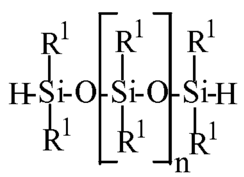

bevorzugt, wobei $R^1$ die oben angegebenen Bedeutung hat und n je nach gewünschter Wiederholungseinheit $V^1$ oder $V^{2*}-Z^2-V^{2*}-$, $n_2$ oder $n_1$ ist, die wie oben definiert sind. Sofern nicht kommerziell erhältlich, können diese Siloxane nach bekannten Verfahren, z.B. durch Äquilibrierung hergestellt werden (Silicone, Chemie und Technologie, Vulkan-Verlag, Essen 1989, S. 82-84).

Die einleitende Einführung der Strukturelemente $V^{2*}$ und Q kann z. B. auf zwei Wegen erfolgen.

Einerseits ist es möglich, zunächst tertiäre Aminofunktionen tragende ungesättigte Strukturen, beispielsweise N,N-Dimethylallylamin, durch Hydrosilylierung direkt an das Siloxan in $\alpha,\omega$-Stellung zu binden. Dieser Prozeß ist allgemein bekannt (B. Marciniec, Comprehensive Handbook on Hydrosilylation, Pergamon Press, Oxford 1992, S. 122-124).

Andererseits ist bevorzugt, durch Hydrosilylierung zunächst reaktive $\alpha,\omega$-funktionalisierte Zwischenprodukte zu erzeugen, welche nachfolgend in $\alpha,\omega$-ditertiäre Aminostrukturen oder direkt in die erfindungsgemäßen quartären

Ammoniumstrukturen umgewandelt werden können. Geeignete Ausgangsstoffe zur Erzeugung reaktiver Zwischenstufen sind beispielsweise halogenierte Alkene oder Alkine, speziell Allylchlorid, Allylbromid, Chlorpropin und Chlorbutin, ungesättigte Halogencarbonsäureester, speziell Chloressigsäureallylester, Chloressigsäurepropargylester, 3-Chlorpropionsäureallylester und 3-Chlorpropionsäurepropargylester und epoxyfunktionelle Alkene, beispielsweise Vinylcyclohexenoxid und Allylglycidether. Die allgemeine Durchführung von Hydrosilylierungen mit Vertretern der genannten Stoffgruppen ist ebenfalls bekannt (B. Marciniec, Comprehensive Handbook on Hydrosilylation, Pergamon Press, Oxford 1992, S. 116-121, 127-130, 134-137, 151-155).

In einem nachfolgenden Schritt können die reaktiven Zwischenstufen dann mit sekundäre Aminofunktionen tragenden Verbindungen zur Reaktion gebracht werden. Geeignete Vertreter sind N,N-Dialkylamine, beispielsweise Dimethylamin, Diethylamin, Dibutylamin, Diethanolamin und N-Methylglucamin, cyclische sekundäre Amine, beispielsweise Morpholin und Piperidin, sekundäre Aminofunktionen tragende Aminoamide, beispielsweise die Umsetzungsprodukte von Diethylentriamin oder Dipropylentriamin mit Lactonen, wie γ-Butyrolacton, Gluconsäure-δ-lacton und Glucopyranosylarabonsäurelacton (DE-OS 4318536, Beispiele 11a, 12a, 13a), oder sekundär-tertiäre Diamine, wie beispielsweise N-Methylpiperazin. Es ist speziell bevorzugt, entsprechende Imidazol- oder Pyrazolderivate, speziell Imidazol und Pyrazol zur Einführung tertiärer Aminofunktionen zu nutzen.

Als Partner für die in einer Ausführungsform bevorzugt eingesetzten Epoxidderivate eignen sich besonders die genannten sekundär-tertiären Diamine, sowie auch Imidazol und Pyrazol. Auf diese Weise können die Alkylierungen regioselektiv und ohne zusätzlichen Aufwand an die Wasserstoffatome tragenden Stickstoffatome dirigiert werden.

Zur Absicherung einer quantitativen Umwandlung der reaktiven Gruppierungen in tertiäre Aminostrukturen werden die Amine in einem Verhältnis von $1 \leq \sum$ sekundäre Aminogruppen : reaktive Gruppen $\leq 10$, bevorzugt 1 bis 3, speziell 1 bis 2, ganz speziell 1 eingesetzt. Aminüberschüsse müssen ggf. entfernt werden.

Die Anbindung der vorstehend beschriebenen α,ω-ditertiären Aminosiloxane an $V^1$ entsprechende Monomer-Einheiten -[$V^1$]- oder eine Präpolymereinheit -[$V^1$-(Q-$V^1$)$_x$]- führt zur Ausbildung von höher alkylierten Amin- bzw. quarternären Ammoniumeinheiten und kann wiederum auf zwei vorteilhaften Wegen erfolgen.

Einerseits ist es bevorzugt, separat ein stark hydrophiles, polyquaternäres, difunktionelles Vorkondensat -[$V^1$-(Q-$V^1$)$_x$]- zu erzeugen, welches zu einem geeigneten Zeitpunkt mit den α,ω-ditertiären Aminosiloxanen vereinigt wird und zum polyquaternären Siloxancopolymeren reagiert.

Die Herstellung hoch geladener, difunktioneller Präpolymere unterschiedlicher Kettenlänge -[$V^1$-(Q-$V^1$)$_x$]- ist beispielhaft in WO 99/14300 (Beispiele 1 bis 7, Tabelle 11) beschrieben. In Abhängigkeit vom molaren Verhältnis von $V^1$ und dem Q zugrunde liegenden Amin kann entweder ein durch Aminogruppen terminiertes oder ein durch andere Reaktivgruppen (Epoxy- bzw. Halogenalkylgruppen) terminiertes Präpolymer erzeugt werden.

Für den Fall der Anbindung eines durch Aminogruppen terminierten Präpolymer -[N-($V^1$-N)$_x$]- an die Aminfunktion einer α,ω-ditertiären Aminosiloxanstruktur kann beispielsweise ein der Wiederholungseinheit $V^1$ entsprechendes, alkylierendes bzw. quaternierendes, difunktionelles Monomer-[$V^1$]-, ausgewählt beispielsweise aus Bisepoxiden, Epichlorhydin, Bishalogenalkyl-Verbindungen, verwendet werden. Es braucht dabei nicht erwähnt zu werden, das unterschiedliche Gruppen $V^1$ im Präpolymer und im Verbindungsglied zwischen Präpolymer und α,ω-ditertiärer Aminosiloxanstruktur resultieren können.

Für den Fall eines durch Reaktivgruppen terminierten Präpolymers, wie -[$V^1$-(Q-$V^1$)$_x$]- kann eine direkte Anbindung an die Aminfunktion der α,ω-ditertiären Aminosiloxanstruktur ohne weiteren Linker erfolgen, da bei der Präpolymersynthese bereits ein Überschuß der $V^1$ erzeugenden Komponente eingesetzt wurde.

Alternativ zur separaten Herstellung eines Vorkondensates -[$V^1$-(Q-$V^1$)$_x$]- kann der Aufbau hoch geladener Blöcke parallel zum Einbau in das Copolymere erfolgen. Dies bedeutet, daß das α,ω-ditertiäre Aminosiloxan mit den Startkomponenten zum Aufbau von -[$V^1$-(Q-$V^1$)$_x$]-, d.h. beispielsweise -[$V^1$]- und Mono oder Diamine der oben erwähnten Bedeutung -[N]- und/oder -[N-N-]- gemeinsam vorgelegt und zur Reaktion gebracht wird.

Schließlich ist es möglich, das α,ω-ditertiäre Aminosiloxan mit langkettiger Siloxaneinheit $Z^2$ oder kurzkettiger Siloxaneinheit $Z^1$ bzw. das α,ω-difunktionelle Siloxan -[$V^{2*}$-$Z^2$-$V^{2*}$]- oder -[$V^1$]- in die vorgelegten Komponenten zum Aufbau von -[$V^1$-(Q-$V^1$)$_x$]- über einen Zeitraum schrittweise zu dosieren oder aber umgekehrt diese Komponenten dem α,ω-ditertiären Aminosiloxan bzw. α,ω-difunktionellen Siloxan schrittweise hinzuzufügen. Eine vorgelagerte Bereitstellung von durch Aminogruppen terminierten Präpolymeren, wie z.B. -[N-($V^1$-N)$_x$]- eröffnet die Möglichkeit, direkt mit geeigneten reaktiven Zwischenstufen, beispielsweise Epoxyderivaten, die Copolymerenbildung auszuführen.

Es ist ebenfalls bevorzugt, die reaktiven Zwischenstufen und die Startkomponenten für den Aufbau von -[$V^1$-(Q-$V^1$)$_x$]- gemeinsam vorzulegen und anschließend zur Reaktion zu bringen.

Schließlich ist möglich, die reaktiven Zwischenstufen in die vorgelegten Komponenten zum Aufbau von -[$V^1$-(Q-$V^1$)$_x$]- über einen Zeitraum schrittweise zu dosieren oder aber umgekehrt diese Komponenten der reaktiven Zwischenstufe schrittweise hinzuzufügen.

Unabhängig von der Wahl eines der vorstehend beschriebenen Reaktionswege und der damit eng verbundenen Frage, ob Aminoeinheiten zunächst das Siloxan oder aber das Präpolymer terminieren, wird die Gesamtstöchiometrie so gewählt, dass die Summe der Aminofunktionen und der mit ihnen reaktionsfähigen Gruppen etwa 1:1 beträgt.

Im Rahmen der Erfindung ist es möglich, von dieser bevorzugten Gesamtstöchiometrie abzuweichen. Es werden dann allerdings Produkte erhalten, die nicht mehr die anvisierte Länge des hoch geladenen, hydrophilen Blocks $-[V^1-(Q-V^1)_x]-$ aufweisen und zusätzlich einen Überschuß einer nicht abreagierten Startkomponenten hinterlassen.

Neben der vorstehend behandelten Gesamtstöchiometrie der Reaktion ist für das Eigenschaftsbild der Produkte die Wahl der die Wiederholungseinheit $V^1$ bildenden Komponente(n) von großer Bedeutung.

Geeignete difunktionelle, den Wiederholungseinheiten $V^1$ zugrunde liegenden Monomere $-[V^1]-$ sind z.B. die Halogencarbonsäureester der Polyalkylenoxiddiole. Bevorzugte Ausgangsmaterialien für deren Synthese sind niedermolekulare, oligomere und polymere Alkylenoxide der allgemeinen Zusammensetzung

$$HO[CH_2CH_2O]_q-[CH_2CH(CH_3)O]_rH$$

wobei q und r die oben angegebenen Bedeutungen aufweisen, und es sich um statististische oder blockartige Einheiten handelt. Bevorzugte Vertreter hinsichtlich des Alkylenoxidblockes sind Ethylenglycol, Diethylenglycol, Triethylenglycol, Tetraethylenglycol, die Oligoethylenglycole mit Molgewichten von 200 bis 10000 g/mol, speziell 300 bis 800, sowie 1,2-Propylenglycol, 1,3-Propylenglycol und Dipropylenglycol.

Die Veresterung der Alkylenoxide erfolgt in an sich bekannter Weise (Organikum, Organisch-chemisches Grundpraktikum, 17. Auflage, VEB Deutscher Verlag der Wissenschaften, Berlin 1988, S. 402-408) durch Reaktion mit den $C_2$- bis $C_4$-Halogencarbonsäuren, deren Anhydriden oder Säurechloriden. Bevorzugt werden die Säurechloride der Chloressigsäure und 3-Chlorpropionsäure eingesetzt und die Reaktion in Abwesenheit von Lösungsmitteln durchgeführt.

In analoger Weise können Alkandiole, Alkendiole und Alkindiole in die entsprechenden reaktiven Esterderivate überführt werden. Bespielhafte Alkohole sind 1,4-Butandiol, 1,6-Hexandiol, 1,4-But(2-)enol und 1,4-But(2-)inol.

Die Einführung von Alkylen-, Alkenylen-, Alkinylen- und Aryleinheiten erfolgt vorzugsweise ausgehend von den entsprechenden Halogeniden, speziell Chloriden und Bromiden. Beispielhafte Vertreter sind 1,6-Dichlorhexan, 1,4-Dichlorbut(2-)en, 1, 4-Dichlorbut(2-)in und 1,4-Bis(chlormethyl)benzol.

Polyalkylenoxideinheiten können ebenfalls über die $\alpha,\omega$-Dihalogenverbindungen eingeführt werden. Diese sind aus den oligomeren und polymeren Alkylenoxiden der allgemeinen Zusammensetzung

$$HO[CH_2CH_2O]_q-[CH_2CH(CH_3)O]_rH$$

wobei q und r die oben angegebenen Bedeutungen aufweisen, beispielsweise durch Chlorierung der Hydroxylgruppen mit $SOCl_2$ zugänglich (Organikum, Organisch-chemisches Grundpraktikum, 17. Auflage, VEB Deutscher Verlag der Wissenschaften, Berlin 1988, S. 189-190).

Mono-, di- oder polyhydroxyfunktionelle Einheiten als Gruppe $V^1$ können ausgehend von Epoxidderivaten eingeführt werden.

Kommerzielle Beispiele sind 1-Chlor-2,3-Epoxypropan, der Glycerol-1,3-bis-glycidylether und Diethylenglycoldiglycidylether und Neopentylglycoldiglycidylether.

Soweit nicht kommerziell verfügbar, können die gewünschten Diepoxide beispielsweise durch Reaktion der entsprechenden Diole mit 1-Chlor-2,3-Epoxypropan unter alkalischen Bedingungen synthetisiert werden.

Es liegt im Rahmen der Erfindung, in die Struktur von $V^1$ Siloxanketten $Z^1$ einzuführen. Hieraus ergibt sich u.a. die Möglichkeit, verschieden lange Siloxanketten für den Aufbau des Gesamtmoleküls zu verwenden. Es ist eine bevorzugte Variante, in $V^1$ Siloxanketten $Z^1$ des Kettenlängenbereichs $n_2$ = 0 bis 19, bevorzugt 0 bis 15, besonders bevorzugt 0 bis 10, speziell 0 bis 5, spezieller 0, einzubauen. Geeignete Startmaterialien zum Einbau sind z.B. die entsprechenden $\alpha,\omega$-Diepoxide oder $\alpha,\omega$-Di(monohalogencarbonsäure)-esterstrukturen.

Bei der Umsetzung von Epoxiden mit primären oder sekundären Aminen ist darauf zu achten, daß für Alkylierungen von tertiären Aminogruppen ein mol H+ pro mol Epoxid/ tertiäres Amin zuzusetzen ist.

Die Wahl geeigneter Amine als Ausgangskomponenten für die Bildung von Q in der Wiederholungseinheit $-[V^1-(Q-V^1)_x]-$ bestimmt ebenfalls in hohem Maße die Molekülstruktur. Die Verwendung ditertiärer Amine (entsprechend -[N-N]-), beispielsweise N,N,N',N'-Tetramethylethylendiamin, N,N,N',N'-Tetramethyltetramethylendiamin, N,N,N',N'-Tetramethylhexamethylendiamin, N,N'-Dimethylpiperazin, führt zu Produkten, in denen jedes Stickstoffatom der Wiederholungseinheit quaterniert ist.

Die Verwendung von sekundär-tertiären Diaminen, beispielsweise N-Methylpiperazin, öffnet den Weg zu Wiederholungseinheiten $-[V^1-(Q-V^1)_x]-$, in denen tertiäre und quartäre Amin- bzw. Ammoniumstrukturen im Verhältnis 1 : 1 vorliegen. Eine teilweise oder vollständige nachträgliche Quaternierung verbliebener tertiärer Aminostrukturen stellt eine bevorzugte Variante zur Einstellung einer gewünschten hohen Dichte der quartären Ammoniumgruppen dar. Die entsprechenden aromatischen Amine Imidazol bzw. Pyrazol führen zu Produkten mit einer delokalisierten Ladung.

Bei Einsatz von primär-tertiären Diaminen, beispielsweise N,N-Dimethylpropylendiamin und 1-(3-Aminopropyl)imidazol, speziell in Kombination mit Diepoxiden, können kammartige Strukturen aufgebaut werden, für die der Quaternierungsgrad während einer abschließenden Alkylierung wählbar ist. Grundsätzlich können die Alkylierungen

auch zu Quaternierungsgraden von durchschnittlich weniger als einer quartären Ammoniumgruppe pro Wiederholungseinheit $-[V^1-(Q-V^1)_x]-$ eingestellt werden. Es ist jedoch bevorzugt, mindestens ein Stickstoffatom pro Wiederholungseinheit zu quaternieren.

Ausgehend von disekundären Aminen, beispielsweise Piperazin, N,N'-Bis(2-hydroxyethyl)-hexamethylendiamin, N,N'-Bis(2-hydroxypropyl)hexamethylendiamin, können grundsätzlich auch Wiederholungseinheiten $-[V^1-(Q-V^1)_x]-$ mit einem durchschnittlichen Gehalt von weniger als einer quartären Ammoniumgruppe synthetisiert werden. Die disekundären Amine liefern hierbei zunächst polytertiär aminomodifizierte Siloxancopolymere oder aber Präpolymere, die in einer abschließenden Reaktion teilweise oder vollständig zu $-[V^1-(Q-V^1)_x]-$ quaterniert werden können. Es ist aber auch in dieser Variante bevorzugt, wenigstens ein Stickstoffatom pro Wiederholungseinheit zu quaternieren.

Als geeignete Quaternierungsagenzien kommen die allgemein bekannten Stoffgruppen wie Alkylhalogenide, Halogencarbonsäureester, Epoxidderivaten in Gegenwart von $H^+$ und Dialkylsulfate, speziell Dimethylsulfat, in Betracht.

Die Herstellung nicht kommerziell verfügbarer disekundärer Amine erfolgt in einer bevorzugten Ausführungsform ausgehend von den entsprechenden diprimären Aminen, beispielsweise Hexamethylendiamin durch Alkylierung mit Epoxiden, wie z.B. Ethylenoxid, Propylenoxid, Isopropylglycidether unter Ausnutzung der unterschiedlichen Reaktionsgeschwindigkeiten primärer und sekundärer Amine.

Es war bereits dargelegt worden, daß im Rahmen der Erfindung die Möglichkeit besteht, Siloxanketten $Z^1$ in die Struktur von $V^1$ einzuführen. Als geeignete Startmaterialien wurden exemplarisch die reaktiven Zwischenstufen $\alpha,\omega$-Diepoxide und $\alpha,\omega$-Di(monohalogencarbonsäure)ester benannt.

Als die aus den Ammoniumgruppen resultierenden positiven Ladungen neutralisierende Anionen $A^-$ kommen bevorzugt die während der Quaternierung gebildeten Ionen, wie Halogenidionen, speziell Chlorid und Bromid, Alkylsulfate, speziell Methosulfat, Carboxylate, speziell Acetat, Propionat, Octanoat, Decanoat, Dodecanoat, Tetradecanoat, Hexadecanoat, Octadecanoat, Oleat, Sufonate, speziell Toluensulfonat in Betracht. Jedoch können durch Ionenaustausch auch andere Anionen eingeführt werden. Zu nennen sind beispielsweise organische Anionen, wie Polyethercarboxylate und Polyethersulfate.

Die Quaternierungsreaktionen werden bevorzugt in Wasser, polaren organischen Lösungsmitteln oder Mischungen beider genannter Komponenten ausgeführt. Geeignet sind z.B. Alkohole, speziell Methanol, Ethanol, i-Propanol und n-Butanol, Glycole, wie Ethylenglycol, Diethylenglycol, Triethylenglycol, die Methyl-, Ethyl- und Butylether der genannten Glycole, 1,2-Propylenglycol und 1,3-Propylenglycol, Ketone, wie Aceton und Methylethylketon, Ester, wie Ethylacetat, Butylacetat und 2-Ethyl-hexylacetat, Ether, wie Tetrahydrofuran und Nitroverbindungen, wie Nitromethan. Die Wahl des Lösungsmittels richtet sich wesentlich nach der Löslichkeit der Reaktionspartner, der angestrebten Reaktionstemperatur und einer gegeebenfalls vorhandenen, die Umsetzung störenden Reaktivität.

Die Reaktionen werden im Bereich von 20°C bis 130°C, vorzugsweise 40°C bis 100°C ausgeführt.

Um die Bildung von gelartigen, nicht vollständig löslichen, linearen Polyorganosiloxanpolymeren zu vermeiden, wird das Molgewicht zweckmäßig nach oben begrenzt.

Eine Begrenzung des Molekulargewichtes wird durch die sich bei der Reaktion zwischen Epoxiden, und im Reaktionssystem gegebenenfalls vorhandenem Wasser bzw. Alkohol entstehende Endstoppung oder alternativ durch die zusätzliche Verwendung von tertiären Aminen, wie Trialkylaminen oder monofunktionellen gegenüber Aminogruppen reaktive Verbindungen bewirkt. D.h., die linearen Polyorganosiloxanpolymere können neben den naturgemäß aus der Umsetzung der monomeren Ausgangsmaterialien resultierenden terminalen Gruppen auch aus monofunktionellen Kettenabbruchsmitteln, wie Trialkylaminen etc. und z.B. daraus resultierende Ammonium-, Amino-, Ether- oder Hydroxy-Endgruppen aufweisen.

Die vorliegende Erfindung betrifft weiterhin die Verwendung der erfindungsgemäßen linearen Polyorganosiloxanpolymere bzw. der nach dem Verfahren der Erfindung erhaltenen linearen Polyorganosiloxanpolymere in kosmetischen Formulierungen, in Waschmitteln oder zur Oberflächenbehandlung von Substraten.

Die erfindungsgemäßen linearen Polyorganosiloxanpolymere, welche in sich die weichmachenden Eigenschaften von Siloxanstrukturen, und die Tendenz von quartären Ammoniumgruppen zur Adsorption an negativ geladenen Festkörperoberflächen vereinen, können mit Erfolg eingesetzt werden in kosmetischen Formulierungen für die Haut- und Haarpflege, in Polituren für die Behandlung und Ausrüstung harter Oberflächen, in Formulierungen zum Trocknen von Automobilen und anderen harten Oberflächen nach maschinellen Wäschen, zur Ausrüstung von Textilen, Textilfasern, Papier, Papierfasern, Papiervliese, einschließlich Faser-, Textil- und Papiervor- und -endbehandlung, Ausrüstung von Papier für den Kosmetik- und Sanitärbereich, besonders permanente hydrophile Weichmacher, als separate Weichmacher nach dem Waschen von Textilien mit anionischen/nichtionogen Detergenzienformulierungen, als Weichmacher in auf anionischen/nichtionogenen Tensiden beruhenden Formulierungen zur Textilwäsche, sowie als Bügelhilfe und als Mittel zur Verhinderung bzw. Rückgängigmachung von Textilverknitterungen.

Die Erfindung betrifft weiterhin Waschmittel-Zusammensetzungen oder kosmetische Zusammensetzungen, enthaltend mindestens eines der erfindungsgemäßen linearen Polyorganosiloxanpolymere zusammen mit mindestens einem weiteren für die Zusammensetzung üblichen Inhaltsstoff.

Die Verwendung der erfindungsgemäßen Polysiloxanderivate führt bei Anwendung im Haarkosmetikbereich

zu günstigen Effekten hinsichtlich Glanz, Fixierung (Halt), Körper, Volumen, Feuchtigkeitsregulierung, Farbretention, Schutz vor Umwelteinflüssen (UV, Salzwasser usw.), Wiederformbarkeit, antistatischen Eigenschaften, Färbbarkeit, Kämmbarkeit etc. D.h. die quartären Polysiloxanverbindungen können bevorzugt in den Kosmetik- und Haarpflegerezepturen gemäss der WO 02-10257 eingesetzt werden.

## Beispiele

### Beispiel 1

1a) 200g (0.0332 mol Epoxygruppen) eines Epoxysiloxans der durchschnittlichen Zusammensetzung

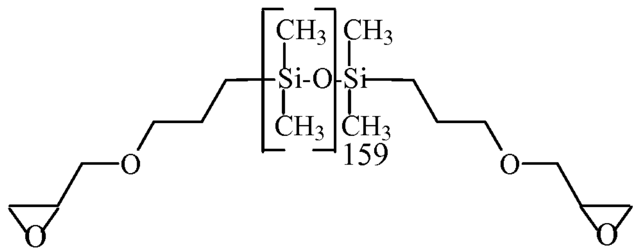

2.26g (0.0332 mol) Imidazol und 100ml 2-Propanol werden in einem mit Stickstoff gespülten Kolben bei Raumtemperatur gemischt und unter Rührung für 9.5 Stunden auf 82-84°C erhitzt. Anschließend werden bei 20hPa/70°C die flüchtigen Bestandteile entfernt. Es werden 196g einer gelben Flüssigkeit der Struktur

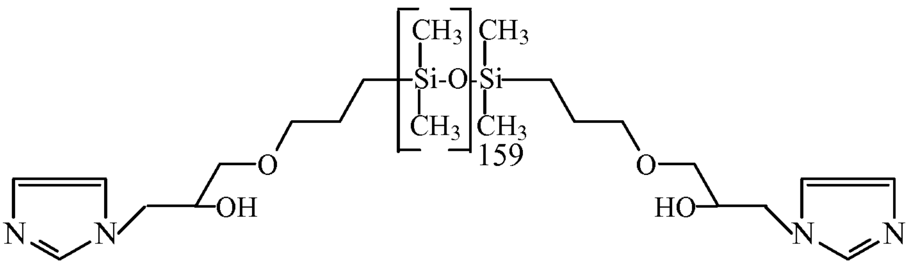

gewonnen, für die im $^1$H-NMR Spektrum die Signale von an Epoxidringen befindlichen Protonen bei 2.6ppm(1H), 2.8ppm(1H) und 3.15ppm(1H) nicht mehr aufgefunden werden können.

1b) 238g (2.24mol) Diethylenglycol werden unter Stickstoff bei Raumtemperatur vorgelegt. Unter intensiver Rührung werden innerhalb einer Stunde 558 g (4,93 mol) Chloressigsäurechlorid zugetropft. Während des Zutropfens steigt die Temperatur auf 82°C an und eine intensive HCl-Entwicklung setzt ein. Nach Beendigung des Zutropfens wird der Ansatz für 30 Minuten auf 130°C erhitzt.

Abschließend werden alle bis 130°C/20hPa siedenden Bestandteile abdestilliert. Es werden 566 g eines hellgelben Öls der Zusammensetzung

$$ClCH_2C(O)OCH_2CH_2OCH_2CH_2OC(O)CH_2Cl$$

erhalten.

Die gaschromatographisch bestimmte Reinheit des Esters beträgt 99,2 %.

$^{13}$C-NMR:

| **Substruktur** | **shift (ppm)** |
|---|---|
| Cl**C**H$_2$- | 40,7 |
| ClCH$_2$-**C**(O)- | 167,1 |
| ClCH$_2$-C(O)-O**C**H$_2$- | 65,2 |
| ClCH$_2$-C(O)-OCH$_2$**C**H$_2$- | 68,6 |

1c) 50g (0.0035mol) des imidazolmodifizierten Siloxans gemäß Beispiel 1a) werden in 60 ml 2-Propanol aufgenommen.

Parallel dazu werden 2.38g (0.035mol) Imidazol, 3.24g (0.035mol) Epichlorhydrin und 0.91g (0.0035mol) des Chloressigsäureesters gemäß Beispiel 1b) jeweils separat in 10 ml 2-Propanol gelöst. Die vier klaren Lösungen werden vereinigt und die Mischung auf 84°C erhitzt. Die Gesamtreaktionszeit beträgt 14.5 Stunden, wobei beginnend nach 1 Stunde eine fortschreitende Trübung des Ansatzes beobachtet wird. Nach Abschluß der Reaktion werden durch Anlegen von Vakuum bis zu 20 hPa alle flüchtigen Bestandteile entfernt. Es werden 46.3g einer weißen, wachsartigen Masse mit einem Feststoffgehalt von 98.3% erhalten. Die folgende Formel zeigt die relative Zusammensetzung:

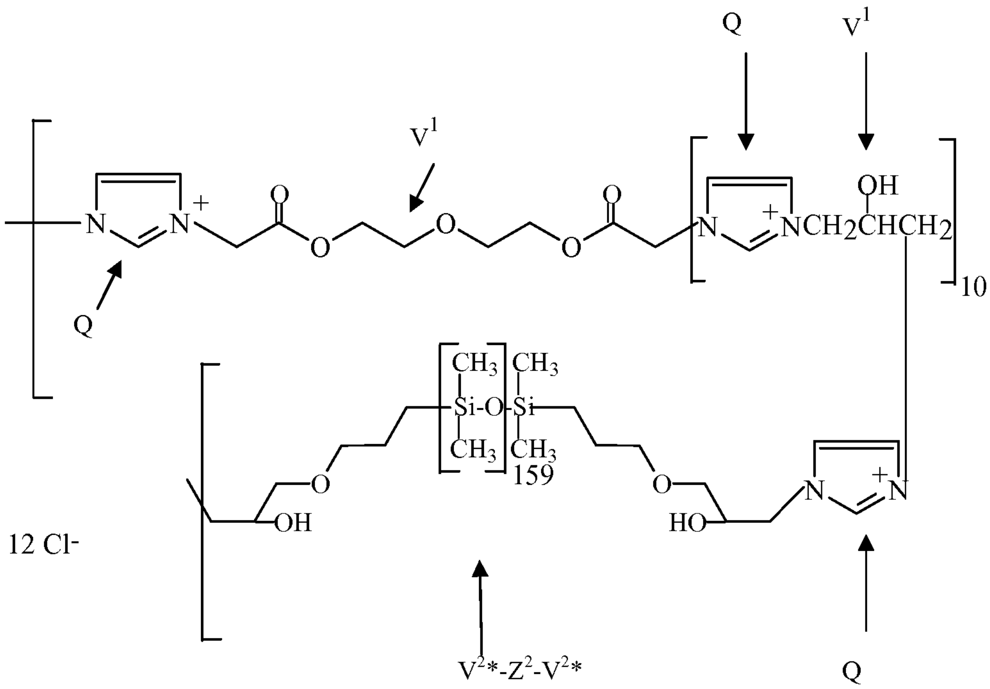

Beispielhaft sind die Wiederholungseinheiten $V^1$, $V^{2*}$-$Z^2$-$V^{2*}$ und Q in die obige Formel eingezeichnet. Das Verhältnis $V^{2*}$-$Z^2$-$V^{2*}$/$V^1$ ist bei diesem Beispiel 1 : 11.

<u>Beispiel 2</u>

2a) 200g (0.0332 mol Epoxygruppen) eines Epoxysiloxans der durchschnittlichen Zusammensetzung

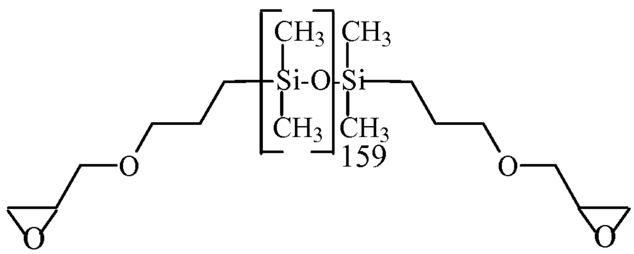

3.33g (0.0332 mol) N-Methylpiperazin und 100ml 2-Propanol werden in einem mit Stickstoff gespülten Kolben bei Raumtemperatur gemischt und unter Rührung für 9.5 Stunden auf 82-84°C erhitzt. Anschließend werden bei 20hPa/70°C die flüchtigen Bestandteile entfernt. Es werden 199g einer gelben Flüssigkeit der Struktur

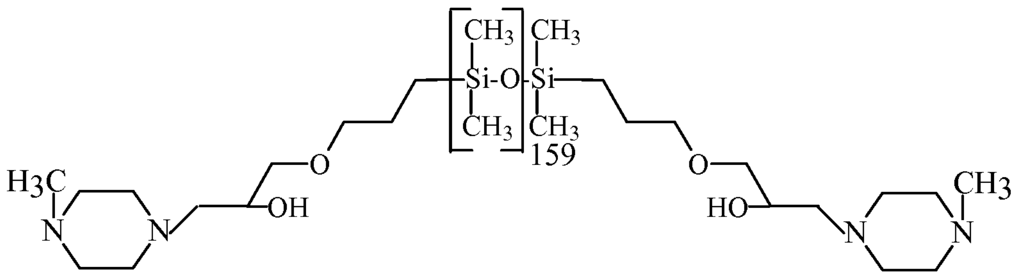

gewonnen, für die im $^1$H-NMR Spektrum die Signale von an Epoxidringen befindlichen Protonen bei 2.6ppm(1H), 2.8ppm(1H) und 3.15ppm(1H) nicht mehr aufgefunden werden können.

2b) 50g (0.00396mol) des durch N-Methylpiperazin modifizierten Siloxans gemäß Beispiel 2a), 4.07g (0.0356mol) N,N'-Dimethylpiperazin und 10.26g (0.0396mol) des Chloressigsäureesters gemäß Beispiel 1b) werden mit 80ml 2-Propanol gemischt und die trübe Lösung für 14.5 Stunden auf 84°C erhitzt. Nach 30 Minuten setzt eine fortschreitende Trübung des Ansatzes ein. Nach Abschluß der Reaktion kann N,N'-Dimethylpiperazin gaschromatographisch nur noch in Spuren aufgefunden werden. Durch Anlegen von Vakuum bis zu 20hPa werden die flüchtigen Bestandteile entfernt. Es werden 61g einer weißen, gummiartigen Masse mit einem Feststoffgehalt von 93.2% erhalten. Die folgende Formel zeigt die quantitative Zusammensetzung:

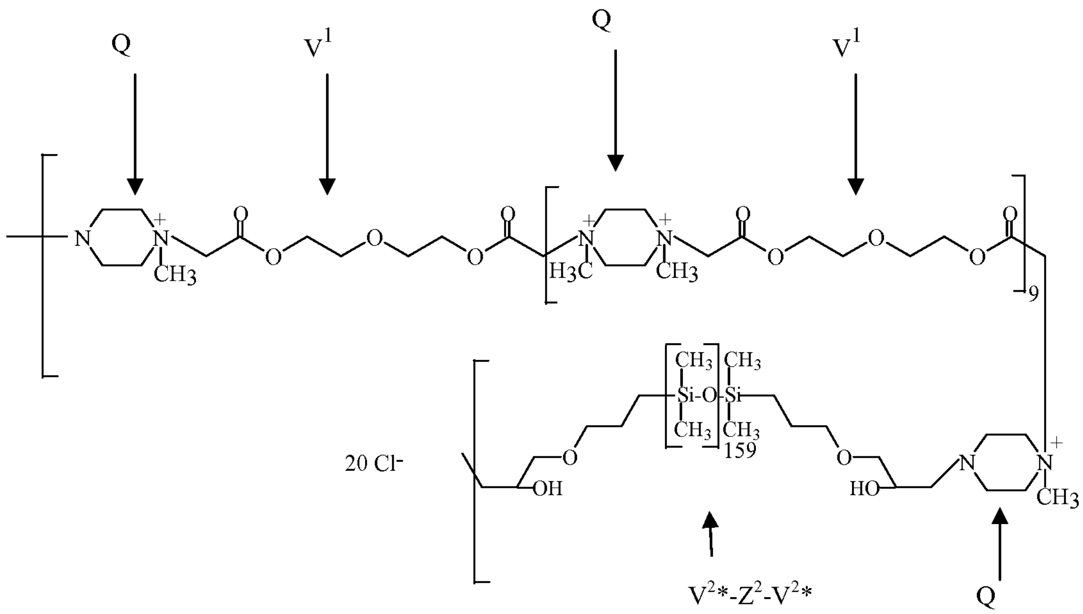

Beispielhaft sind die Wiederholungseinheiten $V^1$, $V^{2*}$-$Z^2$-$V^{2*}$ und Q in die obige Formel eingezeichnet. Das Verhältnis $V^{2*}$-$Z^2$-$V^{2*}$/$V^1$ ist bei diesem Beispiel 1 : 10.

<u>Beispiel 3</u>

19.38g (0.225mol Aminogruppen) N,N,N',N'-Tetramethylhexandiamin und 12.14g (0.202mol) Essigsäure werden mit 30 ml deionisierten Wassers bei Raumtemperatur gemischt. Zu dieser Lösung werden innerhalb 15 Minuten 35.26g (0.202mol Epoxygruppen) einer 50%igen Lösung von Ethylenglycoldiglycidylether in Ethylenglycoldimethylether getropft. Die Temperatur steigt auf 92°C an. Innerhalb einer Nachreaktionszeit von 20 Minuten entsteht eine gelartige Masse. Diese Gelmasse wird einer Mischung zugesetzt, die aus 150g (0.025mol Epoxygruppen) eines Epoxysiloxans der Struktur

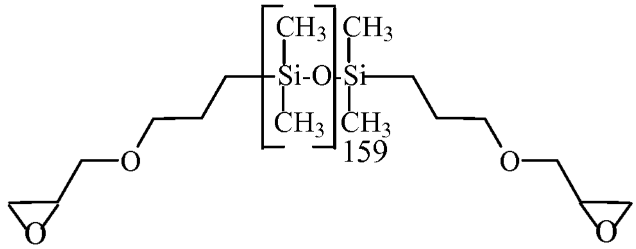

0.75g (0.0125) Essigsäure, 2.5g (0.0125mol) Dodecansäure, 0.33g (0.0025mol; 45%ige wässrige Lösung) Trimethylamin und 50ml 2-Propanol besteht. Die Reaktion erfolgt über 16 Stunden bei 90°C. Anschließend werden bei 20hPa/80°C alle flüchtigen Bestandteile abgezogen. Es werden 182g eines weißen, festen bis wachsartigen Materials erhalten. Die folgende Formel zeigt die quantitative Zusammensetzung:

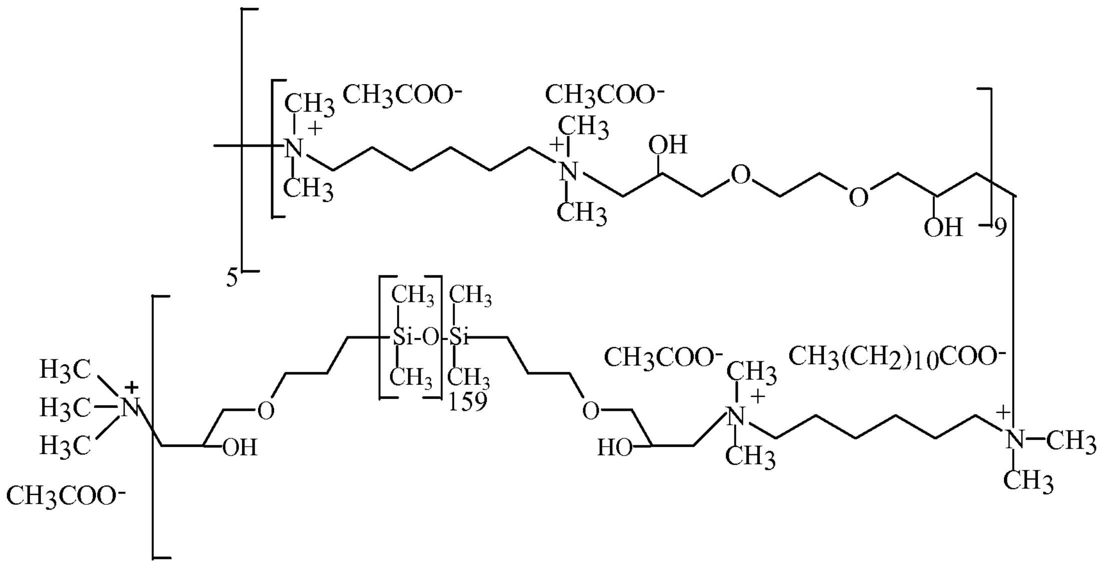

Das Verhältnis $V^{2*}$-$Z^2$-$V^{2*}$/$V^1$ ist bei diesem Beispiel ca. 0,058.

Beispiel 4

27.6g (0.255mol Epoxygruppen) Neopentyldiglycidylether und 54.8g (0.0316mol Epoxygruppen) eines Siloxans der Struktur

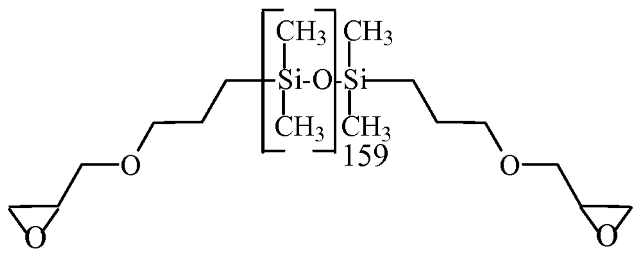

werden bei Raumtemperatur in 200 ml 2-Propanol gelöst. Zu dieser Lösung werden 17.8g (0.142mol primäre Aminogruppen) 1(3-Aminopropyl)imidazol gegeben. Die Ringöffnungsreaktion erfolgt für 8 Stunden bei 80°C. Anschließend werden 17.9g (0.142mol) Dimethylsulfat zugesetzt und innerhalb 5 Stunden die Quaternierungsreaktion durchgeführt. Reste von Dimethylsulfat werden durch Zugabe von 10ml Wasser zersetzt. Nach Abziehen aller bis 20hPa/60°C siedenden Bestandteile werden 97.5g eines braunen, trüben Produktes erhalten. Die folgende Formel zeigt die quantitative Zusammensetzung:

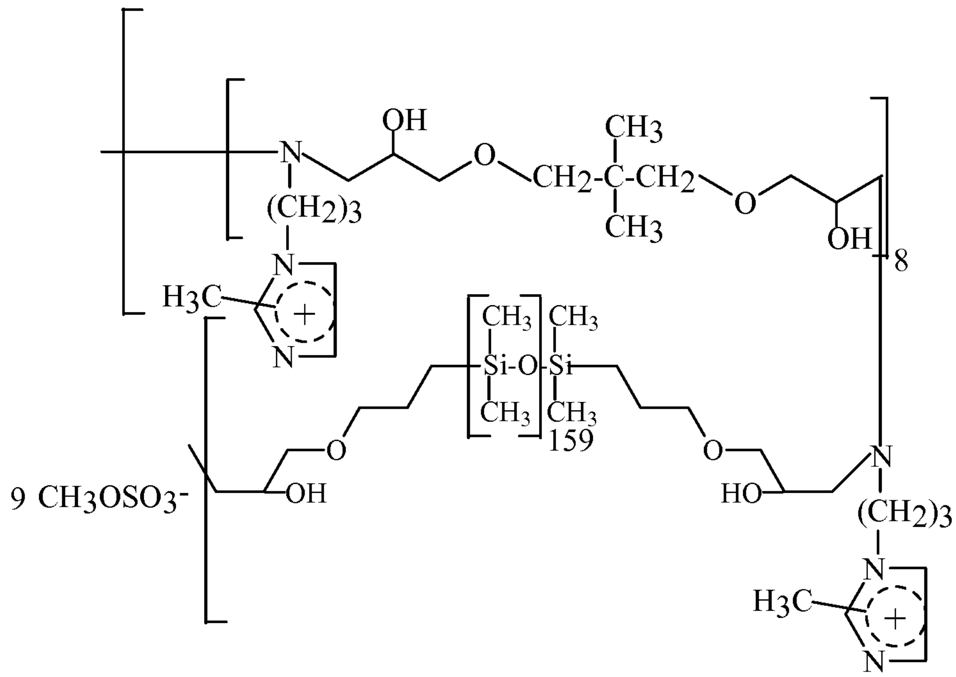

Das Verhältnis $V^{2*}$-$Z^2$-$V^{2*}$/$V^1$ ist bei diesem Beispiel ca. 0,12.

Beispiel 5

9.67g (0.112mol Aminogruppen) N,N,N',N'-Tetramethylhexandiamin, 0,17g (0.0013mol) einer 45%igen wässrigen Trimethylaminlösung, 11,35g (0.056 mol) Dodecansäure und 3.4g (0.056mol) Essigsäure werden mit 6 ml deionisierten Wassers und 124g 2-Propanol bei Raumtemperatur gemischt und auf 50°C erhitzt. In die klare Lösung werden 86.85g (0.0124 mol Epoxygruppen) eines Epoxysiloxans der Struktur

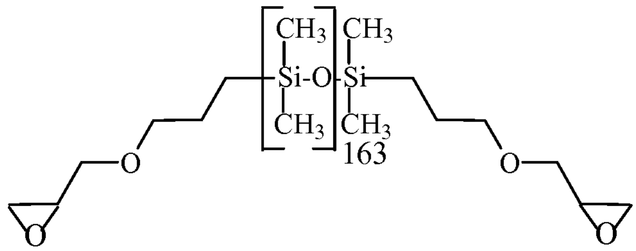

und 18.28g (0.101 mol Epoxygruppen) eines Epoxysiloxans der Struktur

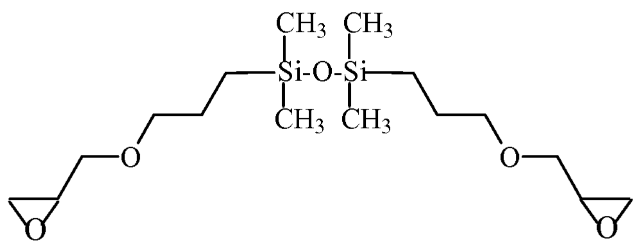

eingetropft. Der Reaktionsansatz wird auf 84°C erhitzt und diese Temperatur 14.5 Stunden aufrecht erhalten. Nach 15 Minuten wurde eine beginnende Eintrübung beobachtet. Nach Abschluß der Reaktion wird der Ansatz geteilt. Aus der einen Hälfte des Ansatzes werden bei 20hPa/80°C alle flüchtigen Bestandteile abgezogen. Es werden 54g einer zähviskosen, fast weißen Masse erhalten. Aus der anderen Ansatzhälfte werden die flüchigen Bestandteile bei 20hPa/25°C entfernt. Es werden 58g eines leicht gelblichen, viskosen Öls gewonnen. Die folgende Formel zeigt die quantitative Zusammensetzung:

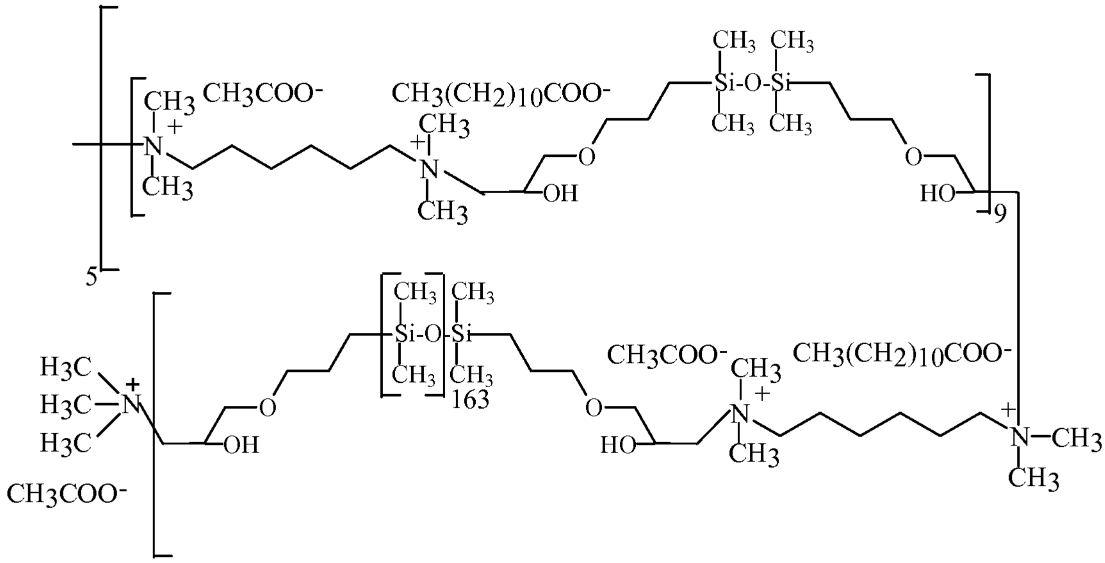

Das Verhältnis $V^{2*}$-$Z^2$-$V^{2*}$/$V^1$ ist bei diesem Beispiel ca. 0,058.

Beispiel 6

Zum Nachweis der weichmachenden Eigenschaften als interner Weichmacher während des Waschprozesses wurden gebleichte und an der Oberfläche nicht weiter ausgerüstete Baumwollstreifen einem Waschprozeß in Gegenwart von Ariel Futur®, bentonithaltigem Dash 2 in 1® (pulverförmig) sowie des in Beispiel 1 beschriebenen Siliconquats unterworfen. Es wurden folgende Randbedingungen eingehalten.

| | Streifen 1 | Streifen 2 | Streifen 3 |
|---|---|---|---|
| Streifengewicht (g) | 13,10 | 12,72 | 13,26 |
| Wassermenge (ml) | 658 | 633 | 669 |
| Detergenz | 0,63g Ariel Futur® | 0,66g Ariel Futur® | 0,67g Dash 2 in 1® |
| Quat.-Bsp. 1 | 0,2 g | - | - |
| Note Ø | 1,2 | 2,9 | 1,9 |

Das Wasser wird auf 60°C erhitzt, die Detergenzien und im Falle des Baumwollstreifens 1 zusätzlich das Siliconquat gemäß Beispiel 1 gelöst. Anschließend werden die Baumwollstreifen in diesen Lösungen für 30 Minuten gewaschen. Nachfolgend werden die Streifen in 5 x 600ml Wasser gespült und abschließend 30 Minuten bei 120°C getrocknet. 12 Testpersonen haben die drei Baumwollstreifen auf die Weichheit des Griffs hin bewertet, wobei die Note 1 dem weichesten Streifen und die Note 3 dem als am härtesten empfundenen Streifen zugeteilt wurde.

Im Ergebnis der Bewertung erhielt der Baumwollstreifen 1 die Durchschnittsnote 1,2. Der Baumwollstreifen 2 wurde durchschnittlich mit 2,9 und der bentonitbehandelte Streifen 3 mit 1,9 bewertet.

**Patentansprüche**

**1.** Lineare Polyamino- und/oder Polyammonium-Polysiloxancopolymere, welche die folgenden Wiederholungseinheiten aufweisen:

$$-[V^{2}*-Z^{2}-V^{2}*-Q]- \text{ und } -[V^{1}-Q]-$$

worin Q aus der Gruppe ausgewählt wird, die besteht aus:

-NR-,

$-N^{+}R_{2}-$

einem gesättigten oder ungesättigten diaminofunktionellen Heterocyclus der Formeln:

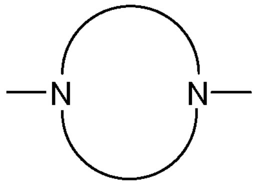

,

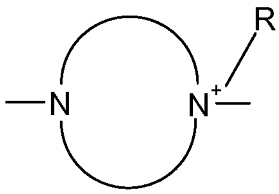

und

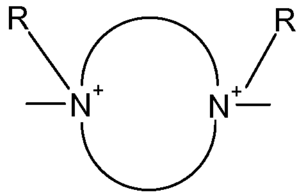

,

sowie

einem aromatischen diaminofunktionellen Heterocyclus der Formel:

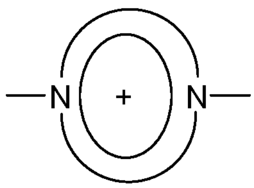

einem dreiwertigen Rest der Formel:

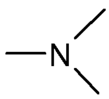

oder

einem dreiwertigen Rest der Formel:

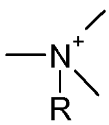

,

worin R jeweils Wasserstoff oder einen einwertigen organischen Rest darstellt,

wobei Q nicht an ein Carbonylkohlenstoffatom bindet,

worin

$V^{2*}$ einen zweiwertigen geradkettigen, cyclischen oder verzweigten, gesättigten, ungesättigten oder aromatischen Kohlenwasserstoffrest mit bis zu 40 Kohlenstoffatomen darstellt, der gegebenenfalls eine oder mehrere Gruppen, ausgewählt aus -O-, -CONH-, $-CONR^2-$, -C(O)- und -C(S)-enthalten kann, und der Rest $V^{2*}$ gegebenenfalls durch eine oder mehrere Hydroxylgruppen substituiert sein kann,
worin $R^2$ = Wasserstoff, ein einwertiger geradkettiger, cyclischer oder verzweigter, gesättigter, ungesättigter $C_1$ bis $C_{16}$ Kohlenwasserstoffrest oder aromatischer $C_6$ bis $C_{16}$ Kohlenwasserstoffrest ist, der eine oder mehrere Gruppen ausgewählt aus -O-, -NH-, -C(O)-, -C(S)- enthalten kann, und der gegebenenfalls durch eine oder mehrere Hydroxylgruppe substituiert sein kann, wobei wenn mehrere Gruppen $-NR^2$ vorliegen, diese gleich oder verschieden sein können,
$-Z^2-$ die Formel

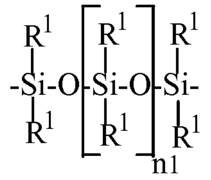

aufweist, worin
$R^1$ gleich oder verschieden sein kann und aus der Gruppe ausgewählt wird, die besteht aus: $C_1$ bis $C_{22}$ Alkyl, Fluor($C_1$-$C_{10}$)alkyl und $C_6$-$C_{10}$ Aryl, und
$n_1$ = 20 bis 1000 bedeutet,
$V^1$ ausgewählt wird aus zweiwertigen oder dreiwertigen, geradkettigen, cyclischen oder verzweigten, gesättigten, ungesättigten oder aromatischen Kohlenwasserstoffresten mit bis zu 1000 Kohlenstoffatomen, die gegebenenfalls eine oder mehrere Gruppen, ausgewählt aus

-O-, -CONH-,
$-CONR^2-$, worin $R^2$ wie oben definiert ist, wobei die Gruppen $R^2$ in den Gruppen $V^1$ und $V^{2*}$ gleich oder verschieden sein können,
-C(O)-, -C(S)- und $-Z^1-$ enthalten können, worin $-Z^1-$ eine Gruppe der Formel

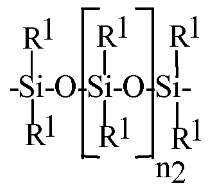

ist, worin
$R^1$ wie oben definiert ist, wobei die Gruppen $R^1$ in den Gruppen $V^1$ und $Z^2$ gleich oder verschieden sein können, und
$n_2$ = 0 bis 19 bedeutet,
und der Rest $V^1$ gegebenenfalls durch eine oder mehrere Hydroxylgruppen substituiert sein kann,

mit der Maßgabe, dass die dreiwertigen Reste Q und die dreiwertigen Reste $V^1$ ausschließlich der Absättigung untereinander innerhalb der linearen Hauptkette der Polysiloxan-Copolymere dienen,
und dass in dem Copolymer das molare Verhältnis

$$-[V^{2}*-Z^{2}-V^{2}*-Q]- \text{ und } -[V^{1}-Q]- < 1:3$$

ist,
und worin die aus den Ammoniumgruppen resultierenden positiven Ladungen durch organische oder anorganische Säureanionen neutralisiert sind,
und deren Säureadditionssalze.

**2.** Lineare Polyamino- und/oder Polyammonium-Polysiloxancopolymere nach Anspruch 1, worin Q aus der Gruppe ausgewählt wird, die besteht aus:

-NR-,
$-N^+R_2-$
einem gesättigten oder ungesättigten diaminofunktionellen Heterocyclus der Formeln:

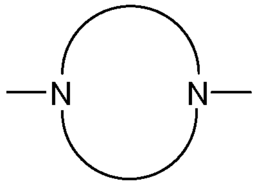
,

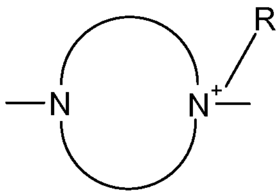

und

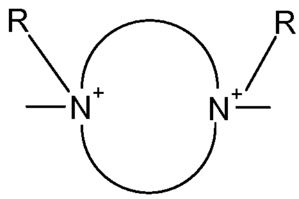
,

sowie
einem aromatischen diaminofunktionellen Heterocyclus der Formel:

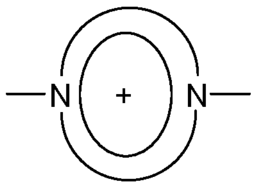

worin R wie oben definiert ist und $V^1$ und $V^{2*}$-$Z^2$-$V^{2*}$ zweiwertige Reste sind.

**3.** Lineare Polyamino- und/oder Polyammonium-Polysiloxancopolymere nach Anspruch 1 oder 2, worin Q aus der Gruppe ausgewählt wird, die besteht aus:

Einer Aminoeinheit der Formel:

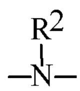

einer Ammoniumeinheit der Formel:

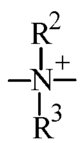

einer quaternierten Imidazoleinheit der Struktur

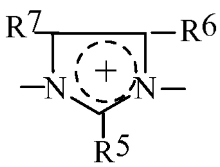

,

einer quaternierten Pyrazoleinheit der Struktur

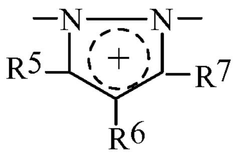

einer zweifach quaternierten Piperazineinheit der Struktur

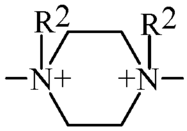

,

einer monoquaternierten Piperazineinheit der Struktur

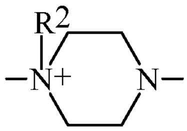

,

einer monoquaternierten Piperazineinheit der Struktur

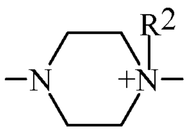

,

einer zweifach quaternierten Einheit der Struktur

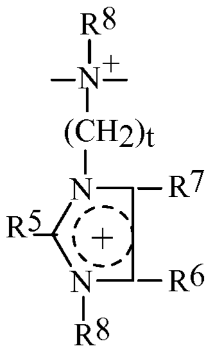

,

einer monoquaternierten Einheit der Struktur

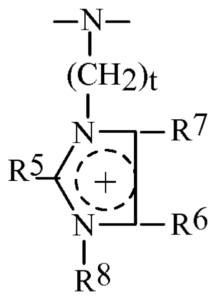

,

einer monoquaternierten Einheit der Struktur

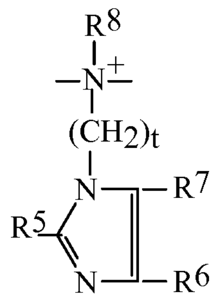

,

einer zweifach quaternierte Einheit der Struktur

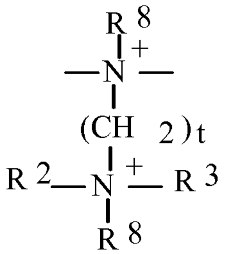

,

einer monoquaternierten Einheit der Struktur

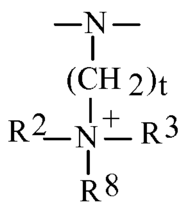

einer monoquaternierten Einheit der Struktur

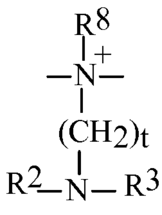

worin

t von 2 bis 10 ist,
$R^2$ wie oben definiert ist, und die Bedeutung von $R^2$ von der Bedeutung der obigen Gruppe $R^2$ gleich oder verschieden sein kann,
$R^3$ die Bedeutung von $R^2$ aufweist, wobei $R^2$ und $R^3$ gleich oder verschieden sein können, oder
$R^2$ und $R^3$ gemeinsam mit dem positiv geladenen Stickstoffatom einen fünf- bis siebengliedrigen Heterocyclus bilden, der gegebenenfalls zusätzlich ein oder mehrere Stickstoff-, Sauerstoff- und/oder Schwefelatome aufweisen kann,
$R^5$, $R^6$, $R^7$ gleich oder verschieden sein können und aus der Gruppe ausgewählt werden, die besteht aus: H, Halogen, Hydroxylgruppe, Nitrogruppe, Cyanogruppe, Thiolgruppe, Carboxylgruppe, Alkylgruppe, Monohydroxyalkylgruppe, Polyhydroxyalkylgruppe, Thioalkylgruppe, Cyanoalkylgruppe, Alkoxygruppe, Acylgruppe, Acetyloxygruppe, Cycloalkylgruppe, Arylgruppe, Alkylarylgruppe, und Gruppen des Typs $-NHR^W$, in denen $R^W$ H, Alkylgruppe, Monohydroxyalkylgruppe, Polyhydroxyalkylgruppe, Acetylgruppe, Ureidogruppe bedeuten, und jeweils zwei der benachbarten Reste $R^5$, $R^6$ und $R^7$ mit den sie an den Heterocyclus bindenden Kohlenstoffatomen aromatische Fünf- bis Siebenringe bilden können, und
$R^8$ die Bedeutung von $R^2$ aufweist, wobei $R^8$ und $R^2$ gleich oder verschieden sein können.

**4.** Lineare Polyamino- und/oder Polyammonium-Polysiloxancopolymere nach einem der Ansprüche 1 bis 3, worin die Gruppe $V^1$ ausgewählt wird aus zweiwertigen, geradkettigen, cyclischen oder verzweigten, gesättigten, ungesättigten oder aromatischen Kohlenwasserstoffresten mit bis zu 600 Kohlenstoffatomen, die gegebenenfalls eine oder mehrere Gruppen, ausgewählt aus
-O-, -CONH-, $-CONR^2-$, worin $R^2$ wie oben definiert ist, -C(O)-, -C(S)- und $-Z^1-$ enthalten kann, worin $-Z^1-$ eine Gruppe der Formel

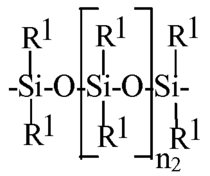

ist, worin

$R^1$ $C_1$ bis $C_3$ Alkyl, Fluor($C_3$-$C_6$)alkyl oder $C_{6-}$ Aryl ist, und
$n_2$ wie oben definiert ist.

**5.** Lineare Polyamino- und/oder Polyammonium-Polysiloxancopolymere nach einem der Ansprüche 1 bis 4, worin die Gruppe Q ausgewählt wird aus

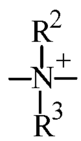

einer quaternierten Imidazoleinheit der Struktur

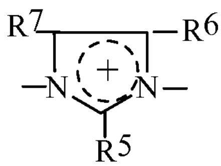

,

einer quaternierten Pyrazoleinheit der Struktur

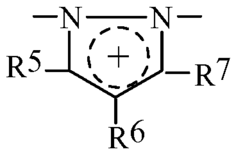

einer zweifach quaternierten Piperazineinheit der Struktur

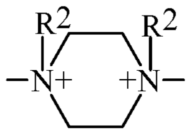

,

einer monoquaternierten Piperazineinheit der Struktur

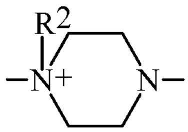

,

einer monoquaternierten Piperazineinheit der Struktur

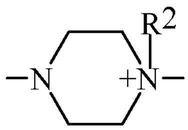

,

einer monoquaternierten Einheit der Struktur

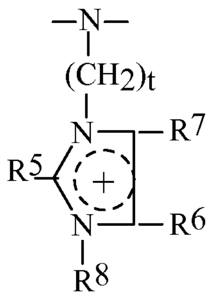

,

worin $R^2$, $R^3$, $R^4$, $R^5$, $R^6$, $R^7$ und $R^8$ wie oben definiert sind.

**6.** Lineare Polyamino- und/oder Polyammonium-Polysiloxancopolymere nach einem der Ansprüche 1 bis 5, worin das molare Verhältnis $-[V^{2*}-Z^2-V^{2*}]-/V^1$ die Beziehung

$$0{,}0005 < -[V^{2}*-Z^{2}-V^{2}*]--/V^{1} < 0{,}3$$

erfüllt.

**7.** Lineare Polyamino- und/oder Polyammonium-Polysiloxancopolymere nach einem der Ansprüche 1 bis 6, worin das molare Verhältnis $-[V^{2*}-Z^2-V^{2*}]-/V^1$ die Beziehung

$$0{,}005 < -[V^{2}*-Z^{2}-V^{2}*]--/V^{1} < 0{,}2$$

erfüllt.

**8.** Verfahren zur Herstellung der linearen Polyamino- und/oder Polyammonium-Polysiloxancopolymere nach einem der Ansprüche 1 bis 7, worin

a) mindestens eine Aminverbindung, ausgewählt aus einer Diamin-Verbindung und/oder einer primären oder sekundären Monoaminverbindung, mit mindestens zwei difunktionellen, zur Reaktion mit den Aminofunktionen der Amin-Verbindung befähigten organischen Verbindungen umgesetzt werden, wobei das molare Verhältnis der organischen Verbindungen so gewählt wird, dass die Bedingung $-[V^{2*}-Z^2-V^{2*}]-/V^1 < 1 : 3$ erfüllt wird, oder
b) mindestens zwei Mol einer Aminverbindung, ausgewählt aus einer Diamin-Verbindung und/oder einer primären oder sekundären Monoaminverbindung, mit einem Mol einer difunktionellen, zur Reaktion mit den Aminofunktionen der Aminverbindung befähigten organischen Verbindung unter Bildung einer Diaminverbindung (Monomer) umgesetzt wird, die anschließend mit mindestens einer Aminverbindung, ausgewählt aus einer Diamin-Verbindung und/oder einer primären oder sekundären Monoaminverbindung, und mindestens einer weiteren difunktionellen zur Reaktion mit den Aminofunktionen der Aminverbindungen befähigten organischen Verbindung umgesetzt wird, oder
c) eine Aminverbindung, ausgewählt aus einer Diamin-Verbindung und/oder einer primären oder sekundären Monoaminverbindung, mit einer difunktionellen, zur Reaktion mit den Aminofunktionen der Aminverbindungen befähigten organischen Verbindung unter Bildung einer Diaminverbindung (aminoterminiertes Oligomer) umgesetzt wird, die anschließend mit mindestens einer difunktionellen zur Reaktion mit den Aminofunktionen der Diamin-Verbindungen befähigten organischen Verbindung umgesetzt wird, oder
d) eine Aminverbindung, ausgewählt aus einer Diamin-Verbindung und/oder einer primären oder sekundären Monoaminverbindung, mit einer difunktionellen, zur Reaktion mit den Aminofunktionen der Aminverbindung befähigten organischen Verbindung unter Bildung einer difunktionellen, zur Reaktion mit Aminofunktionen befähigten Verbindung (difunktionelles Oligomer) umgesetzt wird, die anschließend mit mindestens einer Aminverbindung, ausgewählt aus einer Diamin-Verbindung und/oder einer primären oder sekundären Monoaminverbindung, und mindestens einer weiteren zur Reaktion mit Aminofunktionen befähigten Verbindung umgesetzt wird,

wobei gegebenenfalls monofunktionelle, bevorzugt tertiäre Monoamine oder geeignete, zur Kettenfortpflanzung nicht befähigte Monoamine und/oder monofunktionelle, zur Reaktion mit Aminofunktionen befähigten Verbindungen als Kettenabbruchsmittel hinzugesetzt werden können, und die Stöchiometrie der Aminofunktionen und der zur Reaktion mit Aminofunktionen befähigten funktionellen Gruppen in der letzten Stufe der Umsetzung stets etwa 1:1 beträgt,
und wobei gegebenenfalls vorhandene Aminofunktionen protoniert, alkyliert oder quaterniert werden können.

**9.** Verfahren nach Anspruch 8, worin die funktionellen Gruppen der difunktionellen, zur Reaktion mit Aminofunktionen befähigten Verbindungen ausgewählt werden aus der Gruppe, die besteht aus Epoxygruppen und Halogenalkylgruppen.

**10.** Verwendung der linearen Polyamino- und/oder Polyammonium-Polysiloxancopolymere nach einem der Ansprüche 1 bis 7, sowie der linearen Polyamino- und/oder Polyammonium-Polysiloxancopolymere, die nach Anspruch 8 oder 9 erhalten werden, in kosmetischen Formulierungen, in Waschmitteln oder zur Oberflächenbehandlung von Substraten.

**11.** Verwendung nach Anspruch 10 zur Faserbehandlung bzw. Faserausrüstung.

**12.** Waschmittel-Zusammensetzungen oder kosmetische Zusammensetzungen, enthaltend mindestens ein lineares Polyamino- und/oder Polyammonium-Polysiloxancopolymer nach irgend einem der Ansprüche 1 bis 7 oder mindestens eines der linearen Polyamino- und/oder Polyammonium-Polysiloxancopolymere, die nach einem der Ansprüche 8 oder 9 erhalten werden.

## Claims

**1.** Linear polyamino and/or polyammonium polysiloxane copolymers having the following repeating units:

$-[V^{2*}-Z^2-V^{2*}-Q]-$ and $-[V^1-Q]-$,

wherein Q is selected from the group consisting of:

-NR-,
$-N^+R_2-$
a saturated or unsaturated diaminofunctional heterocycle of the formulas:

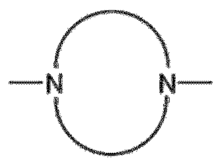

,

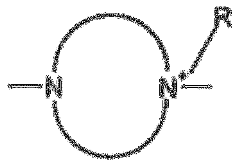

and

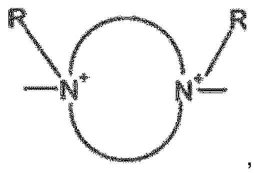

,

as well as
an aromatic diaminofunctional heterocycle of the formula:

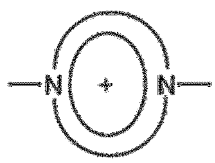

a trivalent residue of the formula:

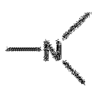

or

a trivalent residue of the formula:

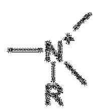

,

wherein R in each case represents a hydrogen or a monovalent organic residue,

wherein Q does not bind to a carbonyl carbon atom,
wherein
$V^{2*}$ represents a divalent straight-chained, cyclic or branched, saturated, unsaturated or aromatic hydrocarbon residue with up to 40 carbon atoms, which can optionally contain one or more groups selected from -O-, -CONH-, $-CONR^2-$, -C(O)- and -C(S)-, and the residue $V^{2*}$ may optionally be substituted with one or more hydroxyl groups, wherein $R^2$ = hydrogen, a monovalent straight-chained, cyclic or branched, saturated, unsaturated $C_1$ to $C_{16}$ hydrocarbon residue, which may contain one or more groups selected from -O-, -NH-, -C(O)-, -C(S)-, and which may be optionally substituted by one or more hydroxyl groups, wherein, when several groups $-NR^2$ are present, these may be the same or different,
$-Z^2-$ has the formula

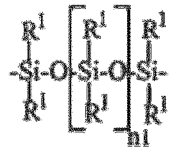

,

wherein
$R^1$ may be the same or different and is selected from the group selected from: $C_1$ to $C_{22}$ alkyl, fluoro($C_1$-$C_{10}$)alkyl and $C_6$-$C_{10}$ aryl, and $n_1$ = 20 to 1000,
$V^1$ is selected from divalent or trivalent, straight-chained, cyclic or branched, saturated, unsaturated or aromatic hydrocarbon residues with up to 1000 carbon atoms, which optionally may contain one or more groups selected from

-O-, -CONH-,
$-CONR^2-$, wherein $R^2$ is as defined above, wherein the groups $R^2$ of the groups $V^1$ and $V^{2*}$ may be the same or different,
-C(O)-, -C(S)- and $-Z^1-$, wherein $-Z^1-$ is a group of the formula

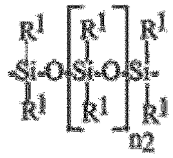

,

wherein $R^1$ is as defined above, wherein the groups $R^1$ in the groups $V^1$ and $Z^2$ may be the same or different, and
$n_2$ = 0 to 19,
and the residue $V^1$ may be optionally be substituted by one or more hydroxyl groups,

with the proviso, that the trivalent residues Q and the trivalent residues $V^1$ exclusively serve the saturation among themselves within the linear main chain of the polysiloxane copolymers,
and that in the copolymer the molar ratio of

$$-[V^{2*}-Z^2-V^{2*}-Q]- \text{ and } -[V^1-Q]- \text{ is } < 1:3,$$

and wherein the positive charges resulting from the ammonium groups are neutralised by organic or inorganic acid anions,
and their acid addition salts.

**2.** Linear polyamino and/or polyammonium polysiloxane copolymers according to claim 1, wherein Q is selected from the group consisting of:

-NR-,
$-N^+R_2$,

an saturated or unsaturated diaminofunctional heterocycle of the formulas:

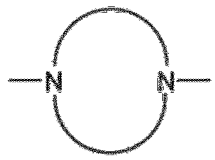

,

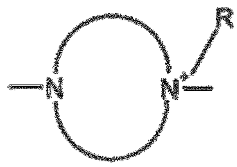

and

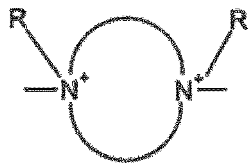

,

as well as
an aromatic diaminofunctional heterocycle of the formula:

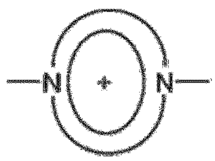

wherein R is as defined above and $V^1$ and $V^{2*}$-$Z^2$-$V^{2*}$ are divalent residues.

**3.** Linear polyamino and/or polyammonium polysiloxane copolymers according to claim 1 or 2, wherein Q is selected from the group consisting of:
An amino unit of the formula:

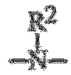

an ammonium unit of the formula

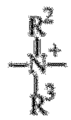

a quaternized imidazole unit of the structure

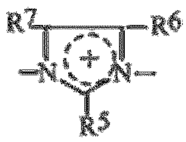

,

a quaternized pyrazol unit of the structure

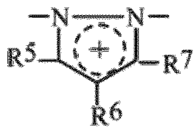

,

a twofold quaternized piperazine unit of the structure

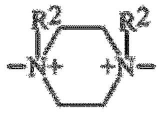

,

a monoquaternized piperazine unit of the structure

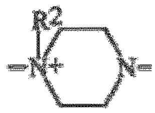

,

a monoquaternized piperazine unit of the structure

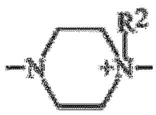

,

a twofold quaternized unit of the structure

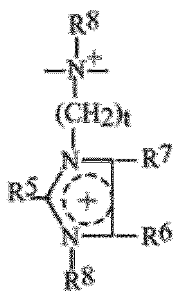

,

a monoquaternized unit of the structure

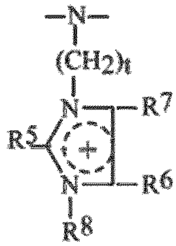

,

a monoquaternized unit of the structure

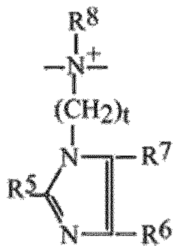

,

a twofold quaternized unit of the structure

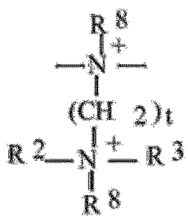

,

a monoquaternized unit of the structure

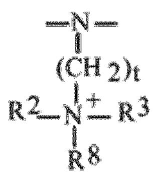

a monoquaternized unit of the structure

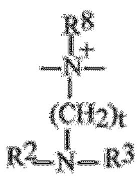

wherein

t is from 2 to 10,
$R^2$ is as defined above, and the meaning of $R^2$ may be the same or different from the meaning of above group $R^2$,
$R^3$ has the meaning of $R^2$, wherein $R^2$ and $R^3$ may be the same or different, or
$R^2$ and $R^3$ form a five- to seven-membered heterocycle together with the positively charged nitrogen atom, which optionally may have one or more nitrogen, oxygen and/or sulfur atoms,

$R^5$, $R^6$; $R^7$ may be the same or different and are selected from the group consisting of: H, halogen, hydroxyl group, nitro group, cyano group, thiol group, carboxyl group, alkyl group, monohydroxy alkyl group, polyhydroxy alkyl group, thioalkyl group, cyano alkyl group, alkoxy group, acyl group, acetyloxy group, cycloalkyl group, aryl group, alkyl aryl group and groups of the type $-NHR^W$, wherein $R^W$ means H, alkyl group, monohydroxy alkyl group, polyhydroxy alkyl group, acetyl group, ureido group, and each two of the vicinal residues $R^5$, $R^6$ and $R^7$ may form aromatic five-to seven-membered rings by the carbon atoms binding them to the heterocycle, and $R^8$ has the meaning of $R^2$, wherein $R^8$ and $R^2$ may be the same or different.

**4.** Linear polyamino and/or polyammonium polysiloxane copolymers according to any of the claims 1 to 3, wherein the group $V^1$ is selected from bivalent, straight-chained or branched, saturated, unsaturated or aromatic hydrocarbon residues with up to 600 carbon atoms, which optionally may contain one or more groups selected from -O-, -CONH-, $-CONR^2-$, wherein $R^2$ is as defined above, -C(O)-, -C(S)- and $-Z^1-$, wherein $-Z^1-$ is a group of the formula

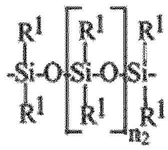
,

wherein

$R^1$ is $C_1$ to $C_3$ alkyl, fluoro($C_3$-$C_6$)alkyl or $C_6$-aryl, and
$n_2$ is as defined above.

**5.** Linear polyamino and/or polyammonium polysiloxane copolymers according to any of the claims 1 to 4, wherein the group Q is selected from

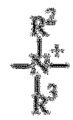

a quaternized imidazole unit of the structure

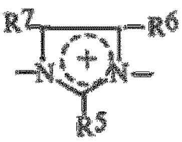
,

a quaternized pyrazole unit of the structure

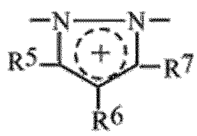

a twofold quaternized piperazine unit of the structure

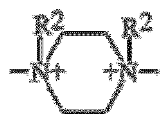
,

a monoquaternized piperazine unit of the structure

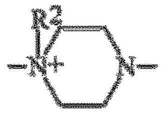

,

a monoquaternized piperazine unit of the structure

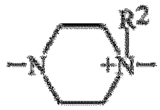

,

a monoquaternized unit of the structure

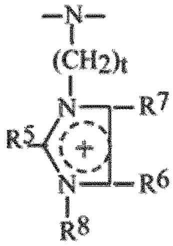

,

wherein $R^2$, $R^3$, $R^4$, $R^5$, $R^6$, $R^7$ and $R^8$ are as defined above.

**6.** Linear polyamino and/or polyammonium polysiloxane copolymers according to any of the claims 1 to 5, wherein the molar ratio $-[V^{2*}-Z^2-V^{2*}]-/V^1$ fulfills the relationship

$$0.0005 < -[V^{2*}-Z^2-V^{2*}]-/V^1 < 0.3 .$$

**7.** Linear polyamino and/or polyammonium polysiloxane copolymers according to any of the claims 1 to 6, wherein the molar ratio $-[V^{2*}-Z^2-V^{2*}]-/V^1$ fulfills the relationship

$$0.005 < -[V^{2*}-Z^2-V^{2*}]-/V^1 < 0.2 .$$

**8.** Process for the production of linear polyamino and/or polyammonium polysiloxane copolymers according to any of claims 1 to 7, wherein

a) at least one amine compound, selected from a diamino compound and/or a primary or secondary monoamino compound, is reacted with at least two difunctional organic compounds being capable of reacting with the amino functions of the amine compound, wherein the molar ratio of the organic compounds is thus selected, that the requirement $< -[V^{2*}-Z^2-V^{2*}]-/V^1 < 1 : 0.3$ is fulfilled, or
b) at least two mole of an amine compound, selected from a diamino compound and/or a primary or secondary monoamino compound, are reacted with one mole of a difunctional organic compound being capable of reacting with the amino functions of the amine compound under formation of a diamine compound (monomer), which is subsequently brought to reaction with at least one amine compound, selected from a diamino compound and/or a primary or secondary monoamino compound, and at least one further difunctional organic compound being capable of reacting with the amino functions of the amine compounds, or
c) an amine compound, selected from a diamine compound and/or a primary or secondary monoamine compound, is reacted with a difunctional organic compound being capable of reacting with the amino functions of the amine compound under formation of a diamine compound (amino-terminated oligomer), which is subsequently brought to reaction with at least one difunctional organic compound being capable of reacting with the amino functions of the diamine compounds, or

d) an amine compound, selected from a diamine compound and/or a primary or secondary monoamine compound, is reacted with a difunctional organic compound being capable of reacting with the amino functions of the amine compound under formation of a difunctional compound being capable of reacting with amino functions (difunctional oligomer), which is subsequently brought to reaction with at least one amine compound, selected from a diamine compound and/or a primary or secondary monoamine compound, and at least one further compound being capable of reacting with amino functions,

wherein optionally monofunctional, preferably tertiary monoamines or suitable monoamines not being capable of chain prolongation and/or monofunctional compounds being capable of reacting with amino functions may be added as chain terminating agent, and the stoichiometry of the amino functions and the functional groups being capable of reacting with the amino functions in the last stage of the reaction is always about 1 : 1,
and wherein optionally present amino functions may be protonated, alkylated or quaternized.

**9.** Process according to claim 8, wherein the functional groups of the difunctional compounds being capable of reacting with amino functions are selected from the group consisting of epoxy groups and halogen alkyl groups.

**10.** Use of linear polyamine and/or polyammonium polysiloxane copolymers according to any of claims 1 to 7 as well as the linear polyamine and/or polyammonium polysiloxane copolymers as obtained according to claims 8 or 9, in cosmetic formulations, in detergents or for the surface treatment of substrates.

**11.** Use according to claim 10 for fiber treatment and fiber finishing, respectively.

**12.** Detergent compositions or cosmetic compositions, containing at least one linear polyamino- and/or polyammonium polysiloxane copolymer according to any of the claims 1 to 7 or at least one linear polyamino- and/or polyammonium polysiloxane copolymer as obtained according to one of the claims 8 or 9.

**Revendications**

**1.** Copolymères polyamino-polysiloxane et/ou polyammonium-polysiloxane linéaires, qui présentent les motifs répétitifs qui suivent :

$$-[V^{2*}-Z^2-V^{2*}-Q]- \text{ et } -[V^1-Q]-$$

dans lesquels Q est choisi parmi le groupe, qui est constitué de :

-NR-
$-N^+R_2-$
un hétérocycle diaminofonctionnel saturé ou insaturé des formules :

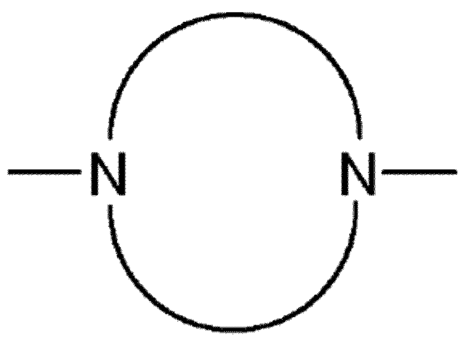

,

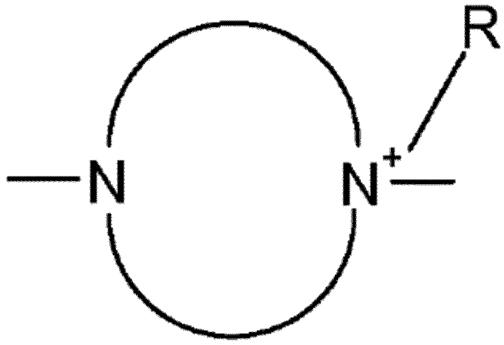

et

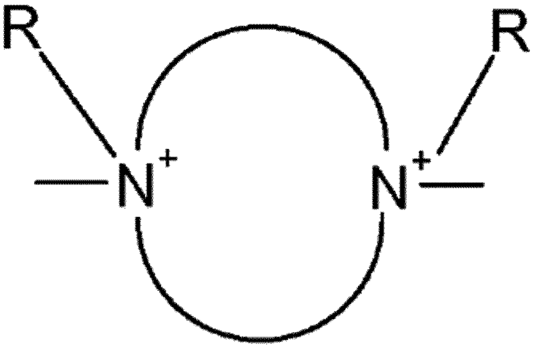

ainsi que
un hétérocycle diaminofonctionnel aromatique de la formule :

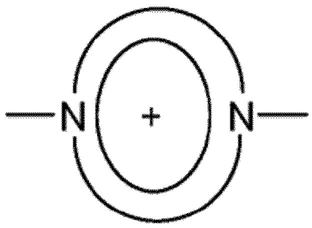

un radical trivalent de la formule :

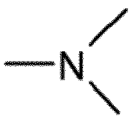

ou

un radical trivalent de la formule :

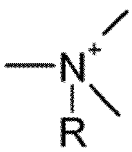

,

dans laquelle R représente respectivement de l'hydrogène ou un radical organique monovalent,
dans lesquels Q ne se lie pas à un atome de carbone de carbonyle,
dans lesquels
$V^{2*}$ représente un radical hydrocarboné bivalent, en chaîne droite, cyclique ou ramifié, saturé, insaturé ou aromatique comprenant jusqu'à 40 atomes de carbone, lequel peut éventuellement contenir un ou plusieurs groupes, choisis parmi -O-, -CONH-, $-CONR^2-$, -C(O)- et -C(S)-, et le radical $V^{2*}$ peut être substitué éventuellement par un ou plusieurs groupes hydroxyle,
dans lesquels $R^2$ = de l'hydrogène, un radical hydrocarboné en $C_1$ à $C_{16}$ monovalent, en chaîne droite, cyclique ou ramifié, saturé, insaturé ou un radical hydrocarboné aromatique en $C_6$ à $C_{16}$, qui peut contenir un ou plusieurs groupes choisis parmi -O-, -NH-, -C(O)-, -C(S)- et qui peut être substitué éventuellement par un ou plusieurs groupes hydroxyle, dans lesquels en présence de plusieurs groupes $-NR^2$, ces derniers peuvent être identiques ou différents,
$-Z^2-$ présente la formule

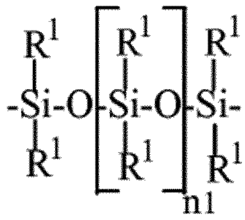

dans laquelle
$R^1$ peut être identique ou différent et est choisi parmi le groupe, qui est constitué de : alkyle en $C_1$ à $C_{22}$, de fluoroalkyle en $C_1$-$C_{10}$ et d'aryle en $C_6$-$C_{10}$, et
$n_1$ = 20 à 1000,
$V^1$ est choisi parmi des radicaux hydrocarbonés bivalents ou trivalents, en chaîne droite, cycliques ou ramifiés, saturés ou insaturés ou aromatiques, comprenant jusqu'à 1000 atomes de carbone, qui peuvent contenir éventuellement un ou plusieurs groupes choisis parmi
-O-, -CONH-,
$-CONR^2-$, dans lequel $R^2$ est défini tel que décrit plus haut, dans lesquels les groupes $R^2$ dans les groupes $V^1$ et $V^{2*}$ peuvent être identiques ou différents,
peuvent contenir -C(O)-, -C(S)- et $-Z^1-$, dans lequel $-Z^1-$ est un groupe de la formule

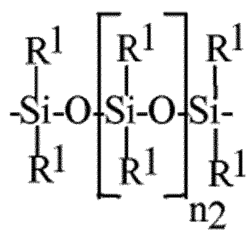
,

dans laquelle
$R^1$ est tel que défini plus haut, dans lesquels les groupes $R^1$ dans les groupes $V^1$ et $Z^2$ peuvent être identiques ou différents, et
$n_2$ = 0 à 19,
et le radical $V^1$ peut être substitué éventuellement par un ou plusieurs groupes hydroxyle,
à condition que les radicaux trivalents Q et les radicaux trivalents $V^1$ servent exclusivement à la saturation entre eux à l'intérieur de la chaîne principale linéaire des copolymères de polysiloxane,
et que dans le copolymère, le rapport molaire

$$-[V^{2*}-Z^2-V^{2*}-Q] \text{ et } -[V^1-Q]- \text{ est } < 1:3,$$

et dans laquelle les charges positives résultant des groupes ammonium sont neutralisées par des anions acides organiques ou inorganiques,
et leurs sels d'addition acides.

**2.** Copolymères polyamino-polysiloxane et/ou polyammonium-polysiloxane linéaires selon la revendication 1, dans lesquels Q est choisi parmi le groupe, qui est constitué :

de -NR-,
de $-N^+R_2-$,
d'un hétérocycle diaminofonctionnel saturé ou insaturé des formules :

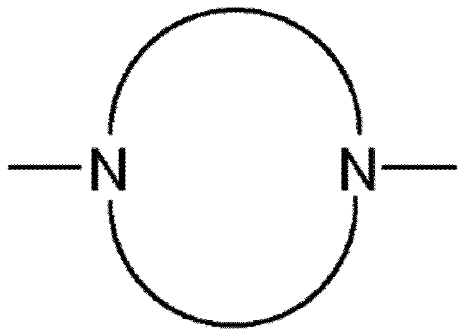

,

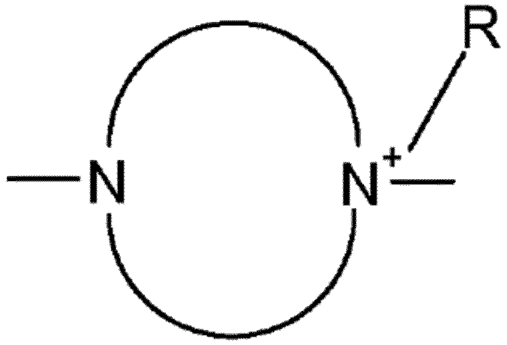

et

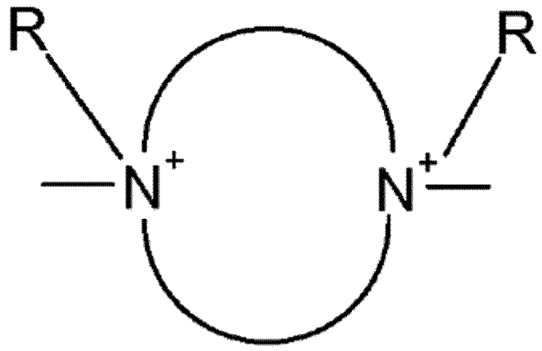

ainsi que
d'un hétérocycle diaminofonctionnel aromatique de la formule :

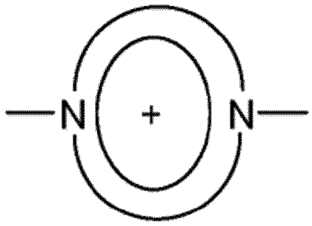

dans laquelle R est tel que défini plus haut et $V^1$ et $V^{2*}$-$Z^2$-$V^{2*}$ sont des radicaux bivalents.

**3.** Copolymères polyamino-polysiloxane et/ou polyammonium-polysiloxane linéaires selon la revendication 1 ou 2, dans lesquels Q est choisi parmi le groupe, qui est constitué de :

un motif amino de la formule :

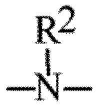

un motif d'ammonium de la formule :

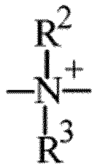

un motif imidazole quaternisé de la structure

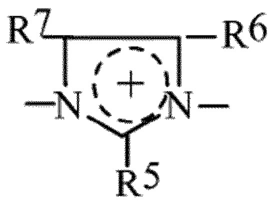

,

un motif pyrazole quaternisé de la structure

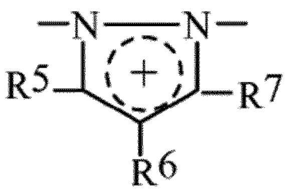

un motif pipérazine doublement quaternisé de la structure

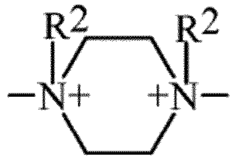

,

un motif pipérazine monoquaternisé de la structure

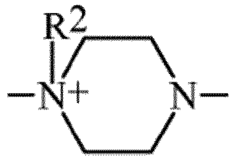

,

un motif pipérazine monoquaternisé de la structure

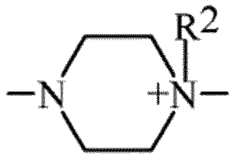

,

un motif doublement quaternisé de la structure

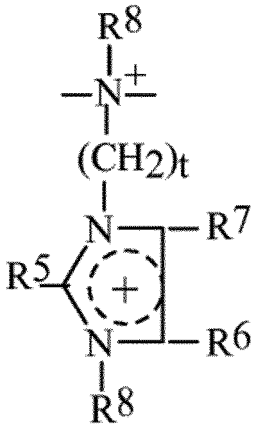

,

un motif monoquaternisé de la structure

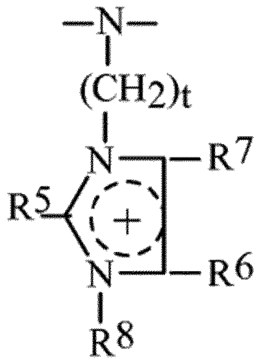

,

un motif monoquaternisé de la structure

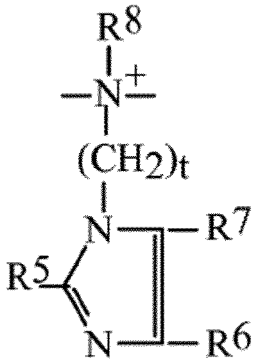

,

un motif doublement quaternisé de la structure

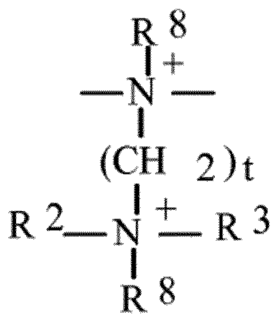

,

un motif monoquaternisé de la structure

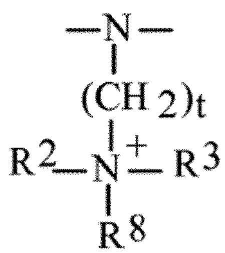

un motif monoquaternisé de la structure

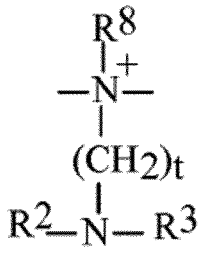

dans lesquelles

t va de 2 à 10,

$R^2$ est défini tel que ci-dessus et la signification de $R^2$ peut être identique ou différente de la signification du groupe ci-dessus $R^2$,

$R^3$ présente la signification de $R^2$, dans lesquels $R^2$ et $R^3$ peuvent être identiques ou différents, ou

$R^2$ et $R^3$ forment conjointement avec l'atome d'azote chargé positivement un hétérocycle à 5 ou 7 membres, qui peut présenter éventuellement en supplément un ou plusieurs atomes d'azote, d'oxygène et/ou de soufre,

$R^5$, $R^6$, $R^7$ peuvent être identiques ou différents et sont choisis parmi le groupe qui est constitué : de H, d'halogène, de groupe hydroxyle, de groupe nitro, de groupe cyano, de groupe thiol, de groupe carboxyle, de groupe alkyle, de groupe monohydroxyalkyle, de groupe polyhydroxyalkyle, de groupe thioalkyle, de groupe cyanoalkyle, de groupe alcoxyle, de groupe acyle, de groupe acétyloxy, de groupe cycloalkyle, de groupe aryle, de groupe alkylaryle et de groupes du type $-NHR^W$, dans lesquels $R^W$ signifient du H, un groupe alkyle, un groupe monohydroxyalkyle, un groupe polyhydroxyalkyle, un groupe acétyle, un groupe uréido, et respectivement deux des radicaux adjacents $R^5$, $R^6$ et $R^7$ peuvent former avec les atomes de carbone les liant à l'hétérocycle, des cycles pentagonaux à heptagonaux aromatiques,

et

$R^8$ présente la signification de $R^2$, dans lesquels $R^8$ et $R^2$ peuvent être identiques ou différents.

**4.** Copolymères polyamino-polysiloxane et/ou polyammonium-polysiloxane linéaires selon l'une quelconque des revendications 1 à 3, dans lesquels le groupe $V^1$ est choisi parmi des radicaux hydrocarbonés bivalents, en chaîne droite, cycliques ou ramifiés, saturés, insaturés ou aromatiques comprenant jusqu'à 600 atomes de carbone, qui peuvent contenir éventuellement un ou plusieurs groupes choisis parmi
-O-, -CONH-, $-CONR^2-$, dans lesquels $R^2$ est tel que défini plus haut, peuvent contenir -C(O)-, -C(S)- et $-Z^1-$, dans lesquels $-Z^1-$ est un groupe de la formule

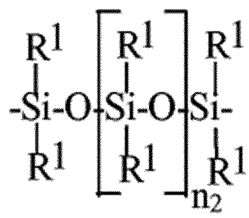

,

dans laquelle

$R^1$ est un alkyle en $C_1$ à $C_3$, un fluoroalkyle en $C_3$-$C_6$ ou un aryle en $C_6$, et
$n_2$ est défini tel plus haut.

**5.** Copolymères polyamino-polysiloxane et/ou polyammonium-polysiloxane linéaires selon l'une quelconque des revendications 1 à 4, dans lesquels le groupe Q est choisi parmi

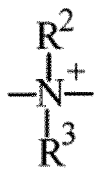

un motif imidazole quaternisé de la structure

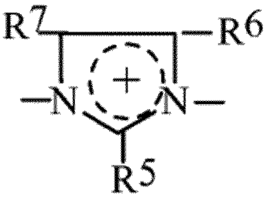

,

un motif pyrazole quaternisé de la structure

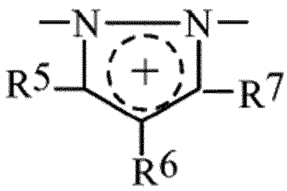

un motif pipérazine doublement quaternisé de la structure

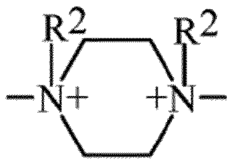

,

un motif pipérazine monoquaternisé de la structure

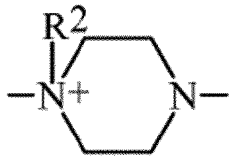

,

un motif pipérazine monoquaternisé de la structure

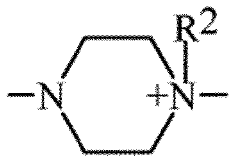

,

un motif monoquaternisé de la structure

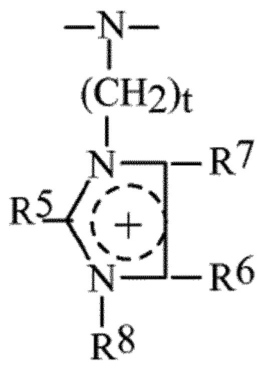

,

dans laquelle $R^2$, $R^3$, $R^4$, $R^5$, $R^6$, $R^7$ et $R^8$ sont tels que définis plus haut.

**6.** Copolymères polyamino-polysiloxane et/ou polyammonium-polysiloxane linéaires selon l'une quelconque des revendications 1 à 5, dans lesquels le rapport molaire $-[V^{2*}-Z^2-V^{2*}]-/V^1$ remplit la relation :

$$0,0005 < -[V^{2*}-Z^2-V^{2*}]--/V^1 < 0,3$$

**7.** Copolymères polyamino-polysiloxane et/ou polyammonium-polysiloxane linéaires selon l'une quelconque des revendications 1 à 6, dans lesquels le rapport molaire $-[V^{2*}-Z^2-V^{2*}]-/V^1$ remplit la relation :

$$0,005 < -[V^{2*}-Z^2-V^{2*}]--/V^1 < 0,2$$

**8.** Procédé servant à fabriquer des copolymères polyamino-polysiloxane et/ou polyammonium-polysiloxane linéaires selon l'une quelconque des revendications 1 à 7, dans lequel

a) au moins un composé amine choisi parmi un composé diamine et/ou un composé monoamine primaire ou secondaire, est mis en réaction avec au moins deux composés organiques difonctionnels, en mesure de réagir avec les fonctions amino du composé amine, dans lesquels le rapport molaire des composés organiques est choisi de telle sorte que la condition $-[V^{2*}-Z^2-V^{2*}]-/V^1 < 1:3$ est remplie, ou
b) au moins deux moles d'un composé amine, choisi parmi un composé diamine et/ou un composé monoamine primaire ou secondaire, sont mises en réaction avec une mole d'un composé organique difonctionnel en mesure de réagir avec des fonctions amino du composé amine en formant un composé diamine (monomère), lequel est mis en réaction par la suite avec au moins un composé amine, choisi parmi un composé diamine et/ou un composé monoamine primaire ou secondaire, et avec au moins avec un autre composé organique difonctionnel en mesure de réagir avec les fonctions amino des composés amine, ou
c) un composé amine, choisi parmi un composé diamine et/ou un composé monoamine primaire ou secondaire, est mis en réaction avec un composé organique difonctionnel en mesure de réagir avec les fonctions amino des composés amine en formant un composé diamine (oligomère terminé par un amino), lequel est mis en réaction par la suite avec au moins un composé organique difonctionnel en mesure de réagir avec les fonctions amino des composés diamine, ou
d) un composé amine, choisi parmi un composé diamine et/ou un composé monoamine primaire ou secondaire, est mis en réaction avec un composé organique difonctionnel en mesure de réagir avec les fonctions amino du composé amine en formant un composé difonctionnel (oligomère difonctionnel) en mesure de réagir avec des fonctions amino, qui est mise en réaction par la suite avec au moins un composé amine, choisi parmi un composé diamine et/ou un composé monoamine primaire ou secondaire, et avec au moins avec un autre composé en mesure de réagir avec des fonctions amino,

dans lesquels éventuellement des monoamines monofonctionnelles, de manière préférée tertiaires ou des monoamines adaptées qui ne sont pas en mesure de reproduire des chaînes et/ou des composés monofonctionnels en mesure de réagir avec des fonctions amino peuvent être ajoutés en tant qu'agents de terminaison de chaîne, et la stoechiométrie des fonctions amino et des groupes fonctionnels en mesure de réagir avec des fonctions amino s'élève lors de la dernière phase de la mise en réaction systématiquement à environ 1:1,

et dans lesquels éventuellement des fonctions amino présentes peuvent être protonées, alkylées ou quaternisées.

**9.** Procédé selon la revendication 8, dans lequel les groupes fonctionnels des composés difonctionnels en mesure de réagir avec des fonctions amino sont choisis parmi le groupe qui est constitué de groupes époxy et de groupes halogénoalkyle.

**10.** Utilisation des copolymères polyamino-polysiloxane et/ou polyammonium-polysiloxane linéaires selon l'une quelconque des revendications 1 à 7 ainsi que des copolymères polyamino-polysiloxane et/ou polyammonium-polysiloxane linéaires qui sont obtenus selon la revendication 8 ou 9, dans des formulations cosmétiques, dans des agents de nettoyage ou aux fins du traitement de surface de substrats.

**11.** Utilisation selon la revendication 10 aux fins du traitement laser ou aux fins du revêtement appliqué à la fibre.

**12.** Compositions d'agent de nettoyage ou compositions cosmétiques contenant au moins un copolymère polyamino-polysiloxane et/ou polyammonium-polysiloxane linéaire selon l'une quelconque des revendications 1 à 7 ou au moins un des copolymères polyamino-polysiloxane et/ou polyammonium-polysiloxane linéaires, qui sont obtenus selon l'une quelconque des revendications 8 ou 9.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 441530 A
- US 5591880 A
- US 5650529 A
- US 5807956 A
- US 5981681 A
- US 5602224 A
- US 5098979 A
- US 5153294 A
- US 5166297 A
- US 6242554 B
- DE PS3719086 C
- EP 282720 A
- US 6240929 B
- WO 0210259 A
- WO 0071806 A
- WO 0071807 A
- US 6211139 B
- WO 9914300 A
- DE OS3236466 A
- WO 0210257 A
- DE OS4318536 A


### In der Beschreibung aufgeführte Nicht-Patentliteratur


- **J.V.GRUBER.** *Colloids and Surfaces B: Biointerfaces,* 2000, vol. 19, 127-135
- **SILICONE.** Chemie und Technologie. Vulkan-Verlag, 1989, 82-84
- **B. MARCINIEC.** Comprehensive Handbook on Hydrosilylation. Pergamon Press, 1992, 122-124
- **B. MARCINIEC.** Comprehensive Handbook on Hydrosilylation. Pergamon Press, 1992, 116-121, 127-130, 134-137, 151-155
- Organikum, Organisch-chemisches Grundpraktikum. VEB Deutscher Verlag der Wissenschaften, 1988, 402-408
- Organikum, Organisch-chemisches Grundpraktikum. VEB Deutscher Verlag der Wissenschaften, 1988, 189-190